# EUROPEAN PATENT APPLICATION

(11) **EP 4 491 628 A1**
(43) Date of publication of application: **15.01.2025**
(21) Application number: 23766985.8
(22) Date of filing: 10.03.2023
(51) Int. Cl.: C07K 7/08, C07K 1/13, C07K 1/16, C07K 16/00

(54) **METHOD FOR PRODUCING FC-CONTAINING MOLECULE MODIFYING REAGENT**

(30) Priority: 11.03.2022 JP 2022037732; 11.03.2022 JP 2022038459
(71) Applicant: Nihon Medi-Physics Co., Ltd., Tokyo 136-0075 (JP); Kagoshima University, Kagoshima-shi, Kagoshima 890-8580 (JP)
(72) Inventor: TONOYA, Gota, Tokyo 136-0075 (JP); SATO, Mikiko, Tokyo 136-0075 (JP); TAKEDA, Takuya, Tokyo 136-0075 (JP); UENO, Atsushi, Suita-shi, Osaka 564-0053 (JP); ITO, Yuji, Kagoshima-shi, Kagoshima 890-8580 (JP); NAKAYAMA, Hiroshi, Kagoshima-shi, Kagoshima 890-8580 (JP)
(74) Representative: TBK
(86) International application number: PCT/JP2023/009425
(87) International publication number: WO 2023/171809

(57) **Abstract**

A method for producing an Fc-containing molecule modifying reagent, including reacting a protein or peptide with a crosslinking agent in the presence of a base having a stronger basicity than pyridine to bond an amino group in the protein or peptide with the crosslinking agent.

## Description

### [Technical Field]

The present invention relates to a method for producing an Fc-containing molecule modifying reagent and a method for producing a conjugate using the Fc-containing molecule modifying reagent.

Antibodies have conventionally been widely used to detect target molecules in various researches and developments, and have become extremely important in industry as detection reagents and diagnostic agents. Also, antibodies are attracting attention as pharmaceutical products for treating diseases in view of their high specificity for the target molecules.

As a site-specific chemical modification to add function without affecting the specificity of the antibody to the target molecule, the present inventors have heretofore reported on techniques for introducing an amino acid capable of binding to a crosslinking agent into a peptide (hereinafter to be also referred to as IgG-binding peptide) that binds specifically or selectively to antibodies, modifying the amino acid with a crosslinking agent to prepare an IgG-binding peptide that is site-specifically modified with the crosslinking agent, and site-specifically modifying antibodies by using the same (Patent Literature 1).

An IgG-binding peptide is a peptide that can specifically bind to the Fc region of IgG. The present inventors have previously developed CCAP (chemical conjugation by affinity peptide) reagents as antibody-labeling reagents that utilize Fc-binding peptides such as Z33 peptide and Z34C peptide. In CCAP reagents, lysine residues, cysteine residues, aspartic acid residues, glutamic acid residues, 2-aminosuberic acid, diaminopropionic acid, arginine residues, or the amino group of the 1-position amino acid present in the Fc-binding peptide are modified with a reactive functional group. The CCAP method, which binds a CCAP reagent to an antibody, is currently considered for application to radiopharmaceuticals and antibody-drug conjugates as a method that can site-specifically modify antibodies by covalent bonds (Patent Literatures 1 to 3).

### [Citation List]

### [Patent Literature]

[Patent Literature 1]
   WO 2017/217347
[Patent Literature 2]
   WO 2016/186206
[Patent Literature 3]
   WO 2018/230257

### [Summary of the Invention]

When producing an IgG-binding peptide site-specifically modified with a crosslinking agent (hereinafter also referred to as an antibody-modification linker), the inventors have found that, in conventional methods such as the method described in Patent Literature 1, when the IgG-binding peptide is reacted with a crosslinking agent, a hydrolyzate of the antibody-modification linker is generated as a by-product, and further that, because the hydrolyzate of the antibody-modification linker has properties similar to those of the antibody-modification linker, it is difficult to separate the two by a purification method such as high performance liquid chromatography.

Therefore, the development of a method for producing an antibody-modification linker has been desired that reduces the generation of hydrolyzates that are difficult to separate, while maintaining the yield of the antibody-modification linker at the same level as that in conventional methods.

Also, the method of producing succinimidyl glutaryl (SG)-modified CCAP reagents, which have been used in the CCAP method to date, by reacting IgG-binding peptides (IgG-BP) or Z33 and Z34C peptides with the crosslinking reagent disuccinimidyl glutarate (DSG), includes several problems. One is that the reaction requires a high temperature (50°C) and a reaction time of two hours, and the other is that the use of a reversed-phase column for purification causes a low recovery rate and renders the method unsuitable for large-scale preparation.

The present inventors have conducted intensive studies in view of the above-mentioned problems, and, in a first embodiment of the present invention, succeeded in reducing the content of a hydrolyzate of an antibody-modification linker, which is a by-product, while maintaining the yield of the antibody-modification linker at the same level as in the conventional methods, by appropriately selecting the reaction conditions (type of base and reaction temperature) when reacting the IgG-binding peptide with a crosslinking agent, and found a production method for obtaining an antibody-modification linker with a higher purity.

A second embodiment of the present invention aims to provide a production method for an antibody-labeling reagent that can obtain an antibody-labeling reagent in a short time and in a high yield.

To solve this problem, the present inventors improved the reaction method and purification method of IgG-binding peptide or Z33 and Z34C peptides and DSG, and constructed a method for obtaining CCAP reagent in a short time and in a high yield.

In the conventional methods, the reaction of IgG-binding peptide (IgG-BP) or Z33 or Z34C peptide with DSG was performed in DMSO containing 1% pyridine at 50°C for 2 hr. Under such conditions, the possibility of side reactions such as racemization of amino acids in peptide was feared, and a method for making the reaction proceed under milder conditions was required. As a result of investigation, the present inventors have found that the reaction is dramatically accelerated by using another base catalyst instead of 1% pyridine. As will be described later, peptide modification is possible with a yield of nearly 100% in only 15 minutes.

Another improvement in the second embodiment of the present invention is a change in the purification method of the SG-modified CCAP reagent. After the reaction of the peptide with DSG, an operation to remove excess DSG is required, and this was conventionally performed using a reversed-phase column. However, reversed-phase columns have problems of low recovery rates and difficulty in scaling up. Therefore, by switching to size exclusion chromatography using a Sephadex G-10 column, steps were omitted and the recovery rate was improved.

The method for producing an Fc-containing molecule modifying reagent according to the present invention includes reacting a protein or peptide with a crosslinking agent in the presence of a base having a stronger basicity than pyridine, thereby binding an amino group in the protein or peptide to the crosslinking agent. In the present invention, the "antibody-modification linker" and the "antibody-labeling reagent" are included in the "Fc-containing molecule modifying reagent".

Another aspect of the present invention provides a method for producing a conjugate including a linker modification step of performing the method for producing an Fc-containing molecule modifying reagent of the present invention to obtain an Fc-containing molecule modifying reagent, and a conjugation step of mixing the aforementioned Fc-containing molecule modifying reagent with an Fc-containing molecule, and reacting the aforementioned Fc-containing molecule modifying reagent with an amino acid at a side chain terminal of an amino acid constituting the aforementioned Fc-containing molecule to obtain a conjugate of the aforementioned Fc-containing molecule modifying reagent and the aforementioned Fc-containing molecule.

The production method according to the first embodiment of the present invention can reduce the content of hydrolyzates of the antibody-modification linker while maintaining the yield of the antibody-modification linker at the same level as in the conventional methods. In other words, an antibody-modification linker can be produced with a high purity.

The production method according to the second embodiment of the present invention can obtain an antibody-labeling reagent in a short time and in a high yield.

### [Brief Description of the Drawings]

[Fig. 1]
   A diagram showing the scheme of the SG-modification of IgGBP-N3-RRR peptide.
[Fig. 2]
   Diagrams showing the results of reversed-phase HPLC analysis of IgGBP-N3-RRR peptide at before (A) and after (B) the SG-modification.
[Fig. 3]
   Diagrams showing the results of size exclusion chromatography (A) and the results of reversed-phase HPLC analysis after purification (B) of SG-modified reactant of IgGBP-N3-RRR peptide.
[Fig. 4]
   A diagram showing the scheme of the SG-modification of Z33-38azide peptide.
[Fig. 5]
   Diagrams showing the results of reversed-phase HPLC analysis of Z33-38azide peptide at before (A) and after (B) the SG-modification.
[Fig. 6]
   Diagrams showing the results of size exclusion chromatography (A) and the results of reversed-phase HPLC analysis after purification (B) of SG-modified reactant of Z33-38 azide peptide.

### [Description of Embodiments]

Unless otherwise specified, the terms used in the present specification can be used with the meanings generally used in the pertinent technique field.

In the present invention, an "Fc-containing molecule" is any molecule that contains the Fc region of an antibody, and includes polypeptides in which the Fc region is fused with other polypeptides, such as antibodies. As the Fc-containing molecule, a full-length antibody may be used, or an antibody fragment or variant may be used.

In the present invention, "Fc" generally refers to a region in an antibody molecule that contains two polypeptide chains consisting of a hinge region or a part thereof, a CH2 domain, and a CH3 domain; however, it is not particularly limited to this and may not contain a hinge region or a part thereof. Fc can be obtained by partially digesting IgG with a protease.

In the following embodiments, the present invention is described mainly using antibodies as an example of Fc-containing molecules. The present invention is not limited to this, and the antibody-modification linker described in the first embodiment can be used as a linker for modifying Fc-containing molecules, and the antibody-labeling reagent described in the second embodiment can be used as a labeling reagent for Fc-containing molecules.

### (First embodiment)

The first embodiment of the present invention provides a method for producing an antibody-modification linker, including reacting an IgG-binding peptide with a crosslinking agent, in which the aforementioned crosslinking agent has a succinimide group capable of binding to the side chain terminal amino group of an amino acid residue constituting the aforementioned IgG-binding peptide, and the aforementioned reaction is performed at room temperature or below in the presence of a tertiary amine having a stronger basicity than pyridine. In the following, the method is also referred to as the method for producing an antibody-modification linker of the first embodiment.

### 1. Method for producing an antibody-modification linker

The first embodiment of the present invention provides a method for producing an IgG-binding peptide (a peptide that specifically or selectively binds to an antibody) that is site-specifically modified with a crosslinking agent. In the first embodiment of the present invention, the IgG-binding peptide that is site-specifically modified with the crosslinking agent is also referred to as an antibody-modification linker.

### <IgG-binding peptide>

The "IgG" used in the first embodiment of the present invention refers to IgG of mammals, for example, primates such as humans and chimpanzees, laboratory animals such as rats, mice, and rabbits, livestock animals such as pigs, cows, horses, sheep, and goats, and pet animals such as dogs and cats, preferably human IgG (IgG1, IgG2, IgG3, or IgG4). The IgG in the first embodiment of the present invention is further preferably human IgG1, IgG2, or IgG4, or rabbit IgG, and particularly preferably human IgG1, IgG2, or IgG4.

In one aspect, the IgG-binding peptide used in the first embodiment of the present invention includes an amino acid sequence consisting of 13 to 17 amino acid residues represented by the following formula I:

(X)₁₋₃-C-(X)₂-H-(Xaa1)-G-(Xaa2)-L-V-W-C-(X)₁₋₃ (I)

wherein each X is independently any amino acid residue other than cysteine,
C is a cysteine residue,
H is a histidine residue,
Xaa1 is a lysine residue,
G is a glycine residue,
Xaa2 is a glutamic acid residue, a glutamine residue, or an asparagine residue,
L is a leucine residue,
V is a valine residue, and
W is a tryptophan residue,
and is capable of binding to human IgG.

In the above-mentioned formula, the notation (X)₁₋₃ at the N-terminus or C-terminus means that 1 to 3 consecutive amino acid residues X, which are independently any amino acid residue other than cysteine (C or Cys), are present, the amino acid residues that constitute the sequence may be the same or different residues, and preferably all three are not the same residue. Similarly, (X)₂ means that any two consecutive amino acid residues X, which are independently any amino acid residue other than cysteine (C or Cys), are present, the amino acid residues that constitute the sequence may be the same or different, but two consecutive amino acid residues are preferably not the same.

The two cysteine residues of the formula I can form a cyclic peptide by disulfide bonding, and are generally disulfide bonded. Alternatively, in the peptide of the formula I, the sulfide groups in the two cysteine residues may be linked by a group represented by the following formula:

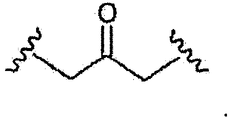

The wavy line portion in the above-mentioned formula means the bond with the sulfide group. This group is more stable against reduction reactions and the like than a normal disulfide bond. Such a peptide can be synthesized, for example, according to the methods described in WO2016/186206 and WO2017/217347.

Specific examples of the IgG-binding peptide of the formula I are listed below in 1) to 18), but it is needless to say that the examples are not limited to these:
1) DCAYH(Xaa1)GELVWCT (SEQ ID NO: 116),
2) GPDCAYH(Xaa1)GELVWCTFH (SEQ ID NO: 117),
3) RCAYH(Xaa1)GELVWCS (SEQ ID NO: 118),
4) GPRCAYH(Xaa1)GELVWCSFH (SEQ ID NO: 119),
5) SPDCAYH(Xaa1)GELVWCTFH (SEQ ID NO: 120),
6) GDDCAYH(Xaa1)GELVWCTFH (SEQ ID NO: 121),
7) GPSCAYH(Xaa1)GELVWCTFH (SEQ ID NO: 122),
8) GPDCAYH(Xaa1)GELVWCSFH (SEQ ID NO: 123),
9) GPDCAYH(Xaa1)GELVWCTHH (SEQ ID NO: 124),
10) GPDCAYH(Xaa1)GELVWCTFY (SEQ ID NO: 125),
11) SPDCAYH(Xaa1)GELVWCTFY (SEQ ID NO: 126),
12) SDDCAYH(Xaa1)GELVWCTFY (SEQ ID NO: 127),
13) RGNCAYH(Xaa1)GQLVWCTYH (SEQ ID NO: 128),
14) G(Xaa3)DCAYH(Xaa1)GELVWCT(Xaa3)H (SEQ ID NO: 129)
15) DCTYH(Xaa1)GNLVWCT (SEQ ID NO: 130),
16) DCAYH(Xaa1)GNLVWCT (SEQ ID NO: 131),
17) DCTYH(Xaa1)GELVWCT (SEQ ID NO: 132), and
18) DCAWH(Xaa1)GELVWCT (SEQ ID NO: 133)
(wherein Xaa1 is a lysine residue, Xaa3 is homocysteine, and preferably, homocysteines form a disulfide bond with each other.)

Specific examples of preferred IgG-binding peptides of the formula I include:
1) DCAYH(Xaa1)GELVWCT (SEQ ID NO: 116),
2) GPDCAYH(Xaa1)GELVWCTFH (SEQ ID NO: 117),
13) RGNCAYH(Xaa1)GQLVWCTYH (SEQ ID NO: 128), and
14) G(Xaa3)DCAYH(Xaa1)GELVWCT(Xaa3)H (SEQ ID NO: 129).
Particularly preferred example thereof is 2) GPDCAYH(Xaa1)GELVWCTFH (SEQ ID NO: 117) wherein Xaa1 is a lysine residue, Xaa2 is homocysteine, and preferably, cysteines and/or homocysteines form a disulfide bond with each other.

The IgG-binding peptide used in the first embodiment of the present invention can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method, and the like. In the synthesis of the peptide, where necessary, the functional groups of the amino acids to be used may be protected. These methods can be performed according to the method described in, for example, WO 2016/186206, WO 2017/217347 and WO 2018/230257.

In the first embodiment of the present invention, the above-mentioned IgG-binding peptide is characterized in that it is modified with a crosslinking agent, and the IgG-binding peptide modified with a crosslinking agent can be used as a linker for modifying an antibody.

When the IgG-binding peptide used in the first embodiment of the present invention is represented by, for example, the formula I, a lysine residue in a specific region of IgG Fc is brought into close proximity in Xaa1. When, for example, IgG is trastuzumab, it is close to Lys248 or Lys246, preferably Lys248, according to EU numbering in human IgG Fc. Therefore, by modifying Xaa1 of the above-mentioned IgG binding peptide with a crosslinking agent, a crosslinking reaction with IgG is performed, and a crosslinking structure can be site-specifically formed between Xaa1 of the IgG binding peptide and the lysine residue of the IgG Fc. By modifying Xaa1 of the IgG-binding peptide with a crosslinking agent and crosslinking same with IgG in this way, various compounds can be specifically and easily introduced into IgG. In addition, since compounds can be introduced via the IgG-binding peptide, compounds with various structures can be introduced into IgG. Furthermore, the method according to the first embodiment of the present invention has the advantage that the yield of the product obtained is high and that since the antibody itself is not modified, the possibility of reducing the function of antibody is low.

In the first embodiment of the present invention, the "crosslinking agent" is a chemical molecule to link the IgG-binding peptide and IgG Fc by a covalent bond. The crosslinking agent in the first embodiment of the present invention can be appropriately selected by those of ordinary skill in the art. Examples thereof include a crosslinking agent preferably containing two or more succinimidyl groups such as DSG (disuccinimidyl glutarate), DSS (disuccinimidyl suberate), and the like, a crosslinking agent preferably containing two or more imide acid moieties such as DMA (dimethyl adipimidate·2HCl, dimethyl adipimidate dihydrochloride), DMP (dimethyl pimelimidate·2HCl, dimethyl pimelimidate dihydrochloride), DMS (dimethyl suberimidate·2HCl, dimethyl suberimidate dihydrochloride), and the like, and a crosslinking agent having an SS bond such as DTBP (dimethyl 3,3'-dithiobispropionimidate·2HCl, dimethyl 3,3'-dithiobispropionimidate dihydrochloride) and DSP (dithiobis(succinimidyl propionate)), and the like. Preferably, the crosslinking agent is one having a succinimide group that can be bonded to the terminal amino group of the side chain of the amino acid residue constituting the IgG-binding peptide, and DSG and DSS are particularly preferred.

In the first embodiment of the present invention, the above-mentioned IgG-binding peptide may be modified with other functional molecules, for example, antibodies such as IgA or VHH, labeling reagents, and/or other drugs. The IgG-binding peptide and other functional substances can be linked by methods known to those skilled in the art, for example, the reaction of an azide group with dibenzocyclooctyne, or the reaction of a maleimide group with a sulfhydryl group. When labeled with a labeling reagent, the IgG-binding peptide forms a conjugate with IgG, making it possible to detect or quantify IgG via the labeling reagent. Labeling reagents include, but are not limited to, fluorescent dyes, chemiluminescent dyes, and fluorescent proteins such as biotin and GFP (green fluorescent protein), luminescent proteins, and enzymes such as peroxidase. Examples of preferred labeling reagents are fluorescein and fluorescein derivatives such as FITC, rhodamine and rhodamine derivatives such as tetramethylrhodamine, and fluorescent dyes such as Texas Red. When the IgG-binding peptide is modified with another drug, examples of the drug include, but are not limited to, anticancer drugs such as auristatin, maytansine, doxorubicin, bleomycin, or derivatives thereof; and targeting agents such as drugs that bind to receptors on the blood-brain barrier to enable migration to the central nervous system, or drugs that bind to cancer cells or the like to enable migration of antibodies into cells.

As one aspect of the first embodiment of the present invention, the N-terminus of the above-mentioned IgG-binding peptide is modified with a chelating agent capable of chelating a radioactive metal or an atomic group capable of a click reaction. In the first embodiment of the present invention, the chelating agent is not particularly limited as long as it has a site in the structure thereof where radioactive metal is coordinated, and has a chelate site, which is a site to which a radioactive metal is coordinated. The chelate site is not particularly limited as long as it has a site in the structure thereof where radioactive metal is coordinated, and preferably has CB-TE2A (1,4,8,11-tetraazabicyclo[6.6.2]hexadecane-4,11-diacetic acid), CDTA (cyclohexane-trans-1,2-diamine tetraacetic acid), CDTPA (4-cyano-4-[[(dodecylthio)thioxomethyl]thio]-pentanoic acid), DOTA (1,4,7,10-tetraazacyclododecane-1,4,7,10-tetraacetic acid), DOTMA ((1R,4R,7R,10R)-α,α',α",α‴-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-carboxyethyl)-1,4,7,10-tetraazacyclododecane-1,4,7,10-tetraacetic acid), DOTP (((1,4,7,10-tetraazacyclododecane-1,4,7,10-tetrayl)tetrakis(methylene))tetraphosphonic acid), DOTMP (1,4,7,10-tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (tetraazacyclododecane dimethanephosphonic acid), Deferoxamine (DFO), DTPA (Glycine, N,N-bis[2-[bis(carboxymethyl)amino]ethyl]-), DTPA-BMA (5,8-bis(carboxymethyl)-11-[2-(methylamino)-2-oxoethyl]-3-oxo-2,5,8,11-tetraazatridecan-13-oic acid), EDTA (2, 2',2",2‴-(ethane-1,2-diylbis(azanetriyl))tetraacetic acid), NOTA (1,4,7-triazacyclononane-1,4,7-triacetic acid), NOTP (1,4,7-triazacyclononane-1,4,7-triyltris(methylenephosphonic acid), TETPA (1,4,8,11-tetraazacyclotetradecane-1,4,8,11-tetrapropionic acid), TETA (1,4,8,11-tetraazacyclotetradecane-N,N',N",N‴-tetraacetic acid), TTHA (3,6,9,12-tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N",N‴-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N",N‴,Nʺʺ-penta-acetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N''-bis(6-carboxy-2-pyridylmethyl)-diethylenetriamine-N,N',N''-triacetic acid), H6phospa (N,N'-(methylenephosphonate)-N,N'-[6-(methoxycarbonyl)pyridin-2-yl]-methyl-1,2-diaminoethane), HP-D03A (hydroxypropyltetraazacyclododecanetriacetic acid), or porphyrin, or a structure derived from a compound represented by the following formula (A). These structures can be appropriately selected according to the type of the below-mentioned radioactive metals.

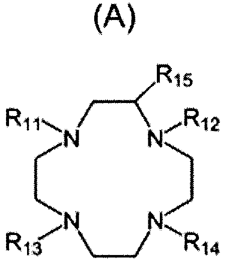

In the formula (A), R₁₁, R₁₃ and R₁₄ are each independently a group consisting of -(CH₂)ₚCOOH, -(CH₂)ₚC₅H₅N, -(CH₂)ₚPO₃H₂, - (CH₂)ₚCONH₂ or -(CHCOOH) (CH₂)ₚCOOH, one of R₁₂ and R₁₅ is a hydrogen atom, a carboxyl group, or a carboxyalkyl group having 2 or 3 carbon atoms, the other is a substituent for binding to the aforementioned IgG-binding peptide, p is an integer of not less than 0 and not more than 3, R₁₅ is a hydrogen atom when R₁₂ is a substituent for binding to the aforementioned IgG-binding peptide, and R₁₅ is a substituent for binding to the aforementioned IgG-binding peptide when R₁₂ is not a substituent for binding to the aforementioned IgG-binding peptide.

Examples of the specific structure represented by the formula (A) include structures derived from the compounds represented by the following formulas (A-1) to (A-12).

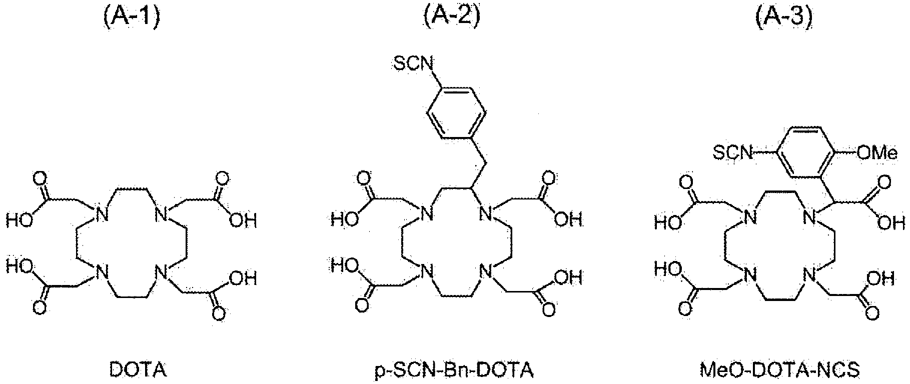

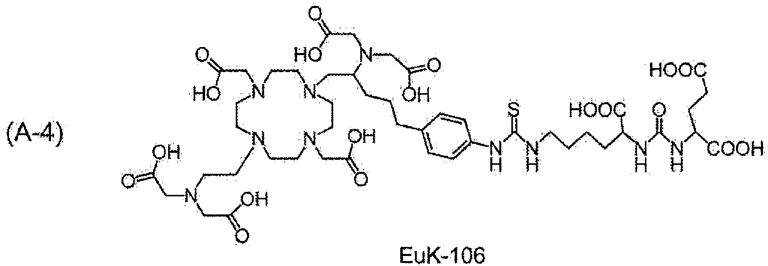

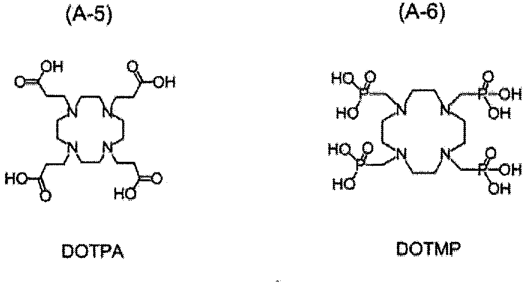

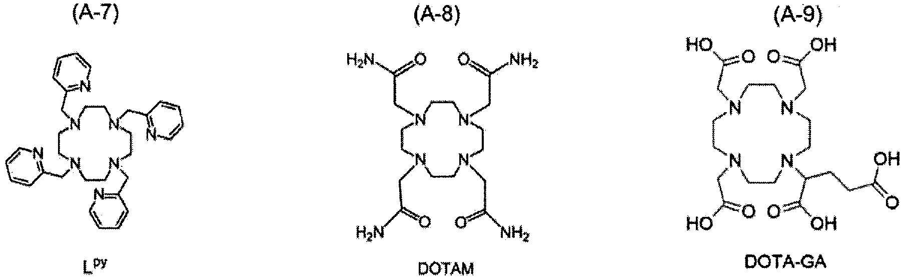

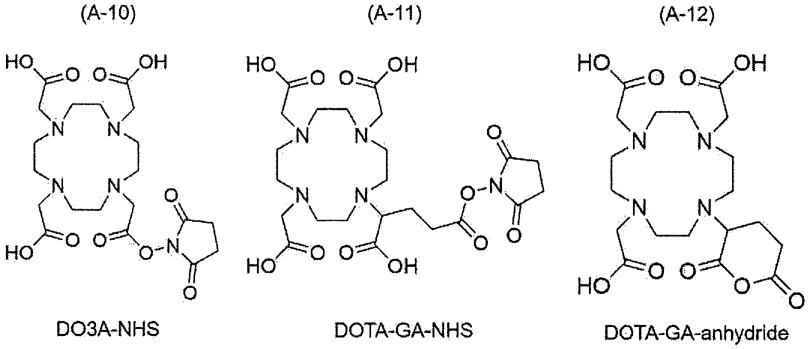

Methods for linking a chelating agent to an IgG-binding peptide are well known to those skilled in the art. For example, when a chelating agent is linked to the N-terminus of an IgG-binding peptide, a reactive group such as N-hydroxysuccinimide ester (NHS), isothiocyano group (ITC), sulfonic acid chloride, carboxylic acid chloride, ethylene oxide, alkyl chloride, aldehyde group, or carboxylic acid anhydride may be added to the chelating agent and reacted with the amino group at the N-terminus of the IgG-binding peptide. Alternatively, an IgG-binding peptide containing such a chelating agent may be directly synthesized by a well-known synthesis method.

As the radioactive metal, a metal nuclide that emits α-ray, β-ray, γ-ray, or a combination thereof can be used. Examples of such radioactive metal nuclide include radioisotope of alkali metal, alkaline earth metal, lanthanoid, actinide, transition metal, and metals other than these metals. Of these, from the aspect of being commercially available and improving chelate formation, ⁴⁴Sc, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th or ²²⁵Ac is preferably used as a radioactive metal nuclide. These radioactive metals can be produced according to a conventional method, and are preferably obtained as a solution containing the radioactive metal in an ionized state.

A person skilled in the art can appropriately select a preferred combination of a radioactive metal and a chelating agent.

As the atomic group capable of click reaction, an appropriate one is selected depending on the type of click reaction. The click reaction can be conducted using, for example, a combination of alkyne and azide, a combination of 1,2,4,5-tetrazine and alkene, and the like. Among these combinations, one atomic group may be introduced into the N-terminus of the IgG-binding peptide, and the other atomic group may be introduced into the chelating agent. Specific examples of the click reaction by such combinations of atomic groups include a Husgen cycloaddition, an inverse electron-demand Diels-Alder reaction, and the like.

Specific examples of the combination of the atomic groups capable of click reaction include, as shown in the following formulas, a combination of an atomic group containing dibenzylcyclooctyne (DBCO) as alkyne (the formula (1a)) and an atomic group containing an azide group as azide (the formula (2a)), or else a combination of an atomic group containing 1,2,4,5-tetrazine (the formula (1b)) and an atomic group containing trans-cyclooctene (TCO) as alkene (the formula (2b)).

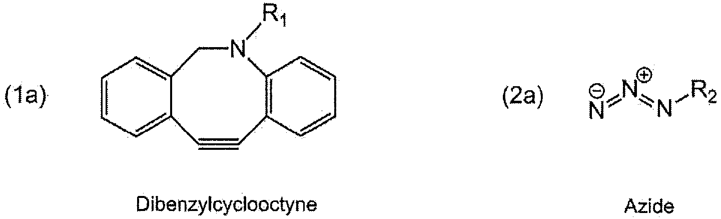

In the formula (1a), R₁ is a binding site with an antibody-modification linker or a chelating agent, and in the formula (2a), R₂ is a binding site with an antibody-modification linker or a chelating agent.

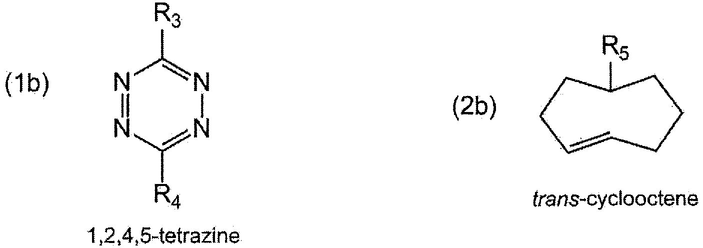

In the formula (1b), one of R₃ and R₄ is a binding site with an antibody-modification linker or a chelating agent, and the other is a hydrogen atom, a methyl group, a phenyl group or a pyridyl group, and in the formula (2b), R₅ is a binding site with an antibody-modification linker or a chelating agent.

Methods for linking an atomic group capable of click reaction to an IgG-binding peptide are well known to those skilled in the art. For example, an introduction method including dissolving IgG-binding peptide in a solution containing a solubilizing agent and a reducing agent and where necessary, an acid, adding an organic solvent solution of an atomic group containing an azide group or trans-cyclooctene (TCO) as the atomic group capable of click reaction to the solution, and stirring the mixture at room temperature can be mentioned.

When an atomic group containing an azide group is introduced as the atomic group capable of click reaction, the azide group is directly introduced into the N-terminus of a peptide by using a commercially available azide group-introducing reagent according to a conventional method. Examples of the azide group-introducing reagent to be used include silyl azide, azide phosphate, alkyl ammonium azide, inorganic azide, sulfonyl azide, PEG azide, and the like.

When an atomic group containing TCO is introduced as the atomic group capable of click reaction, TCO is introduced into the N-terminal of a peptide by using a commercially available click chemistry reagent containing TCO according to a conventional method.

When alkyne is used as an atomic group capable of click reaction and, for example, an atomic group containing dibenzyl cyclooctyne (DBCO) represented by the above-mentioned formula (1a) is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

The aforementioned IgG-binding peptide may be modified, for example, by PEGylation (polyethylene glycol addition) of the N-terminus and amidation of the C-terminus in order to improve its stability. The number of PEG molecules used in PEGylation is not particularly limited, and for example, 1 to 50 molecules, 1 to 20 molecules, 2 to 10 molecules, 2 to 6 molecules, or 4 molecules of PEG can be added.

An IgG-binding peptide modified with a crosslinking agent, i.e., an antibody-modification linker, can be produced, for example, by reacting an IgG-binding peptide obtained according to the method described above in the <IgG-binding peptide> section with a crosslinking agent. This can be achieved by selecting a combination of the type of amino acid at a specific position in the IgG-binding peptide to be modified by the crosslinking agent and the crosslinking agent. For example, a crosslinking agent containing a succinimidyl group such as DSS or DSG reacts with primary amine present in the side chain of lysine residue and the N-terminal of polypeptide. Therefore, only the side chain of the lysine residue can be specifically modified with DSS or DSG by blocking the N-terminal of the IgG-binding peptide and reacting same with DSS or DSG.

The reaction between the IgG-binding peptide and the crosslinking agent is performed within a range that allows the yield of the IgG-binding peptide site-specifically modified with the crosslinking agent, i.e., the antibody-modification linker, to be maintained while reducing the by-product, the hydrolyzate of the antibody-modification linker. Specifically, the IgG-binding peptide is reacted with the crosslinking agent in a solvent in the presence of a "tertiary amine having a stronger basicity than pyridine" as a base at a reaction temperature below room temperature. The solvent used in this reaction is not particularly limited as long as it does not adversely affect the reaction. For example, water, saline, or buffers such as sodium acetate buffer, ammonium acetate buffer, phosphate buffer, phosphate-buffered saline, Tris buffer, HEPES buffer, or tetramethylammonium acetate buffer, or water-soluble organic solvents such as alcohols with 1 to 5 carbon atoms, acetonitrile, N,N-dimethylformamide, tetrahydrofuran, dimethyl sulfoxide, and acetone, or mixed solvents of these can be used.

The "basicity" in this embodiment can be expressed by the pKa of the conjugate acid when the tertiary amine used in the first embodiment of the present invention is used as the base.

The "tertiary amine having a stronger basicity than pyridine" is preferably a tertiary amine having a pKa of 6.0 or more (pyridine has a pKa of 5.25). When the basicity is weak, the reaction does not proceed sufficiently. The use of a strongly basic tertiary amine is expected to result in sufficient reaction, but from the viewpoint of increasing versatility, the pKa is preferably 15.0 or less, and more preferably 8.0 or less. The "tertiary amine having a stronger basicity than pyridine" used in the present invention is preferably a pKa of 6.0 or more and 15.0 or less, and more preferably a pKa of 6.0 or more and 8.0 or less. Specifically, a tertiary alkylamine is preferred, and a tertiary alkylamine having 1 to 10 carbon atoms is more preferred, and may be a chain alkylamine or a cyclic alkyne amine. Examples of the alkylamine include chain alkylamines such as trimethylamine, triethylamine, diethylmethylamine, methyldipropylamine, methyldiisopropylamine, ethyldimethylamine, dimethylpropylamine, ethylmethylpropylamine, and ethylmethylisopropylamine, and cyclic alkylamines such as methylpyrrolidine, ethylpyrrolidine, propylpyrrolidine, isopropylpyrrolidine, butylpyrrolidine, tert-butylpyrrolidine, methylhomopiperidine, ethylhomopiperidine, methylpiperidine, ethylpiperidine, dimethylpiperazine, diethylpiperazine, methylethylpiperazine, N-methylmorpholine, and ethylmorpholine. N-methylmorpholine (NMM) is preferred.

The amount of the tertiary amine, which has a stronger basicity than pyridine, in this reaction is not particularly limited as long as it can promote the reaction. Based on the molar amount of the IgG-binding peptide, the upper limit can be, for example, 20 equivalents or less, 10 equivalents or less, 8 equivalents or less, or 4 equivalents or less, and the lower limit can be, for example, 1 equivalent or more, 2 equivalents or more, 3 equivalents or more, or 4 equivalents or more, and preferably 2 equivalents or more and 20 equivalents or less, 4 equivalents or more and 20 equivalents or less, 2 equivalents or more and 10 equivalents or less, 4 equivalents or more and 10 equivalents or less, 2 equivalents or more and 8 equivalents or less, 4 equivalents or more and 8 equivalents or less, or 2 equivalents or more and 4 equivalents or less. When the amount is too small, the reaction does not proceed sufficiently, and when the amount is too large, the amount of hydrolyzate, which is a by-product, increases.

The amount of the crosslinking agent used in this reaction is not particularly limited as long as it can site-specifically modify the IgG-binding peptide. It is generally an excess amount, and preferably based on the molar amount of the IgG-binding peptide, the upper limit can be, for example, 100 equivalents or less, 50 equivalents or less, 40 equivalents or less, 30 equivalents or less, 20 equivalents or less, 15 equivalents or less, or 10 equivalents or less, and the lower limit can be, for example, 5 equivalents or more, 10 equivalents or more, 20 equivalents or more, 25 equivalents or more, or 30 equivalents or more. When the amount is too small, the reaction does not proceed sufficiently. Even when a certain amount or more of the crosslinking agent is used, the yield reaches a plateau, and therefore, it is preferable to use 20 equivalents or more and 50 equivalents or less, 20 equivalents or more and 40 equivalents or less, or 20 equivalents or more and 30 equivalents or less.

The reaction temperature in this reaction is, for example, room temperature or less. Here, the "room temperature" refers to the temperature defined in the Japanese Pharmacopoeia, 16th Edition, and is generally 1 to 30°C. The reaction temperature can be, for example, 30°C or less, 20°C or less, or 10°C or less, more preferably 5°C or less, further more preferably -5°C or less. This can further suppress the generation of hydrolyzates of the antibody-modification linker as by-products. In addition, in order to suppress an increase in the required amount of tertiary amine having a stronger basicity than pyridine, and/or to prevent the reaction time from becoming too long, the reaction temperature is preferably -40°C or more, more preferably -20°C or more.

The reaction time in this reaction is not particularly limited as long as the reaction proceeds sufficiently, and varies depending on the type and amount of the IgG-binding peptide, crosslinking agent, and tertiary amine (base) to be used, and the reaction temperature, and is set appropriately depending on these. The reaction time is generally 250 min or less, 200 min or less, 180 min or less, 150 min or less, 120 min or less, or 60 min or less. At higher reaction temperatures, the amount of base is smaller and the reaction time is shorter. At lower reaction temperatures, the amount of base is larger and the reaction time is longer.

After completion of the reaction, the reaction solution may be used as is in the conjugation step described below, or may be concentrated or purified by removing the solvent by freeze-drying or the like. Purification conditions are not particularly limited, but for example, ethyl acetate, isopropyl acetate, acetonitrile, or tetrahydrofuran may be used to precipitate a solid, or various chromatography methods such as ultrafiltration, gel filtration chromatography, ion exchange chromatography, affinity chromatography, reversed-phase chromatography, and high performance liquid chromatography may be used. When using reversed-phase chromatography, the mobile phase is preferably a mixture of an organic solvent and an acidic aqueous solution. A mixture of the organic solvent such as acetonitrile, ethanol, methanol, and 2-propanol, and the acidic aqueous solution such as an aqueous solution containing formic acid, acetic acid, or trifluoroacetic acid is exemplified, and a mixture of acetonitrile and an aqueous solution containing trifluoroacetic acid is more preferred. When storing the antibody-modification linker, storing at room temperature or below is preferred, and at 0°C or below is more preferred.

### 2. Method for producing antibody conjugate

As another aspect, the first embodiment of the present invention provides a method for producing an antibody conjugate, including a linker modification step of obtaining an antibody-modification linker by the above-mentioned method (1. Method for producing an antibody-modification linker), and a conjugation step of mixing the antibody-modification linker with an antibody and reacting the succinimide group of the antibody-modification linker with the side chain terminal amino group of the amino acid residue constituting the antibody to obtain a conjugate of the antibody-modification linker and the antibody. In the antibody conjugate, one or more antibody-modification linker molecules only need to be linked to one antibody molecule, and it includes, for example, an antibody conjugate in which one antibody-modification linker molecule is linked to one antibody molecule, or an antibody conjugate in which two antibody-modification linker molecules are linked to one antibody molecule.

The conditions for the conjugation step are not particularly limited as long as they allow a crosslinking reaction to occur between the succinimide group of the crosslinking agent of the antibody-modification linker and the side chain terminal amino group of the amino acid residue constituting the antibody. For example, a solution containing an antibody-modification linker that has undergone a linker modification step may be mixed with a solution containing an antibody, or a solution containing an antibody may be mixed with a solution containing an antibody-modification linker after solvent replacement with a desired buffer solution. A known method can be appropriately adopted for the solvent replacement. The reaction temperature of the conjugation step is not particularly limited as long as it is a temperature at which the antibody-modification linker and the antibody are linked by a crosslinking reaction. The reaction is preferably performed under conditions in which the antibody can exist stably, and more preferably at a temperature at which the antibody-modification linker is not decomposed and the hydrolysate of the antibody-modification linker does not increase excessively. For example, the conjugation step can be performed at room temperature (1°C to 30°C) or normal temperature (15°C to 25°C). The conjugation step is preferably performed under conditions in which the antibody can exist stably, and is preferably performed under weakly acidic conditions of pH 5 to 7. As the solvent for the conjugation step, a buffer solution such as acetate buffer, 2-amino-2-(hydroxymethyl)propane-1,3-diol (Tris) buffer, 2-[4-(2-hydroxyethyl)-1-piperazinyl]-ethanesulfonic acid (HEPES) buffer, phosphate buffer, or histidine buffer can be used. Where necessary, a known catalyst for promoting the crosslinking reaction, a known stabilizer such as arginine, or a sugar such as sucrose or an tonicity agent such as sodium chloride or potassium chloride may be added in an appropriate amount.

The mixing ratio of the antibody-modification linker and the antibody in the conjugation step is not particularly limited. For example, it is 1:1 or more and 20:1 or less, in a molar ratio. The lower limit is preferably 2:1 or more, more preferably 5:1 or more, and the upper limit is preferably 10:1 or less. The reaction time for the conjugation step is not particularly limited, and is, for example, 1 min or more and 5 hr or less. The lower limit is preferably 10 min or more, more preferably 15 min or more, and the upper limit is preferably 2 hr or less, more preferably 1 hr or less.

In the method for producing the antibody conjugate, after the conjugation step, a purification step of the obtained antibody conjugate may be performed as necessary to separate impurities such as unreacted IgG-binding peptide, antibody-modification linker, antibody, and the like, to purify the antibody conjugate. In this case, the purification conditions are not particularly limited. For example, purification methods known in the art such as ultrafiltration, gel filtration chromatography, ion exchange chromatography, affinity chromatography, reversed-phase chromatography, hydrophobic chromatography, and high performance liquid chromatography can be adopted, and affinity chromatography can be preferably used. As the mobile phase, phosphate buffer, acetate buffer, citrate buffer, glycine buffer, and the like can be used, and salts such as ammonium sulfate, sodium chloride, and potassium chloride may be added as appropriate.

The first embodiment of the present invention encompasses the following technical ideas.
[1] A method for producing an antibody-modification linker by reacting an IgG-binding peptide with a crosslinking agent, wherein the aforementioned crosslinking agent has a succinimide group capable of binding to a terminal amino group of a side chain of an amino acid residue constituting the aforementioned IgG-binding peptide, and the aforementioned reaction is performed at room temperature or a lower temperature in the presence of a tertiary amine having a stronger basicity than pyridine.
[2] The method for producing an antibody-modification linker of the above-mentioned [1], wherein the aforementioned tertiary amine is a tertiary amine having a pKa of 6.0 or more and 15.0 or less.
[3] The method for producing an antibody-modification linker of the above-mentioned [2], wherein the aforementioned tertiary amine is a tertiary amine having a pKa of 6.0 or more and 8.0 or less.
[4] The method for producing an antibody-modification linker of the above-mentioned [1] to [3], wherein the aforementioned reaction is performed in the presence of a molar amount of the aforementioned tertiary amine of 2 equivalents to 20 equivalents based on the molar amount of the aforementioned IgG-binding peptide.
[5] The method for producing an antibody-modification linker of the above-mentioned [1] to [4], wherein the aforementioned reaction is performed at 5°C or lower.
[6] The method for producing an antibody-modification linker of the above-mentioned [1] to [4], wherein the aforementioned reaction is performed at -5°C or below.
[7] The method for producing an antibody-modification linker of the above-mentioned [1] to [6], wherein the aforementioned crosslinking agent is DSG (disuccinimidyl glutarate) or DSS (disuccinimidyl suberate).
[8] The method for producing an antibody-modification linker of the above-mentioned [1] to [7], wherein the aforementioned IgG-binding peptide comprises an amino acid sequence consisting of 13 to 17 amino acid residues and represented by the following formula I:

   (X)₁₋₃-C-(X)₂-H-(Xaa1)-G-(Xaa2)-L-V-W-C-(X)₁₋₃ (I)
   wherein each X is independently any amino acid residue other than cysteine,
   C is a cysteine residue,
   H is a histidine residue,
   Xaa1 is a lysine residue,
   G is a glycine residue,
   Xaa2 is a glutamic acid residue, glutamine residue or asparagine residue,
   L is a leucine residue,
   V is a valine residue, and
   W is a tryptophan residue.
[9] A method for producing an antibody-modification linker of the above-mentioned [1] to [8], wherein the N-terminus of the aforementioned IgG-binding peptide is modified with a chelating agent capable of chelating a radioactive metal or an atomic group capable of a click reaction.
[10] A method for producing an antibody conjugate, comprising a linker modification step of performing the method for producing an antibody-modification linker of the above-mentioned [1] to [9] to obtain an antibody-modification linker; and a conjugation step of mixing the aforementioned antibody-modification linker with an antibody and reacting the succinimide group of the aforementioned antibody-modification linker with a terminal amino group of a side chain of an amino acid constituting the aforementioned antibody to obtain a conjugate of the aforementioned antibody-modification linker and the aforementioned antibody.

### (Second embodiment)

The method for producing an antibody-labeling reagent according to the present embodiment includes reacting a protein or peptide with the aforementioned crosslinking reagent (crosslinking agent) in the presence of a base having a stronger degree of basicity than pyridine to bind an amino group in the aforementioned protein or peptide to the crosslinking reagent.

The "degree of basicity" in this embodiment is synonymous with the "basicity" defined in the first embodiment. The base used in this embodiment is a base with a pKa of more than 5.2 (or 5.19, 5.23, or 5.25). Preferably, a base with a pKa of less than 14 (or 14.0) can be used. Alternatively, a base with a pKa of 11 or less can be used. More preferably, a base within the range combining these maximum and minimum values can be used. Examples of such bases include triethylamine (pKa = 10.75), N,N-diisopropylethylamine, diethylamine, and monomethylamine, and triethylamine is preferred. In this embodiment, the pKa is pKa at room temperature (for example, 25°C) .

The concentration of the base is not particularly limited as long as it is a concentration that can bond the amino group and the crosslinking reagent, and can be set appropriately depending on the type of base, protein or peptide, and crosslinking reagent to be used, and the concentration of the protein or peptide and crosslinking reagent. It is preferably 0.1 to 5%, more preferably 0.5 to 3%, 0.8 to 2%, 0.9 to 1.5%, or 1%.

The solvent to be used is not particularly limited as long as it does not affect the reaction and can dissolve the protein or peptide and the crosslinking reagent. It is preferably an aprotic polar solvent, such as dimethyl sulfoxide (DMSO), N,N-dimethylformamide (DMF), chloroform, dichloromethane, diethyl ether, tetrahydrofuran (THF), ethyl acetate, acetone, and acetonitrile, more preferably dimethyl sulfoxide (DMSO) or N,N-dimethylformamide (DMF).

The protein or peptide is preferably a peptide capable of specifically binding to the Fc region of IgG (Fc-binding peptide). "IgG" may be IgG of mammals, for example, primates such as human, chimpanzee and the like, experimental animals such as rat, mouse, rabbit and the like, domestic animals such as swine, bovine, horse, sheep, goat and the like, and pet animals such as dog and cat and the like, and human IgG (IgG1, IgG2, IgG3 or IgG4) is preferred. As IgG, human IgG1, IgG2, or IgG4, or rabbit IgG is preferred and human IgG1, IgG2, or IgG4 is particularly preferred. "IgG Fc region" typically refers to a C-terminal fragment of IgG obtained by treating the IgG with protease papain.

The Fc-binding peptide is preferably a peptide that binds to a site selected from Lys248, Lys246, Lys338, Lys288, Lys290, Lys360, Lys414, and Lys439 and/or adjacent region thereof according to Eu numbering in Fc, preferably Lys248 and/or adjacent region thereof, or that binds to the binding region of Protein A. For example, the Fc-binding peptide may be a partial peptide of Protein A or a variant thereof having Fc binding ability. Specific examples of such peptides are described in International Patent Publications WO2008/054030, WO2013/027796, WO2016/186206, and WO2018/230257, and Kyohei Muguruma et al., ACS Omega (2019); 4(11):14390-14397. They can be appropriately prepared according to the methods described in each document.

Examples of such peptides include the following peptides (i) to (iii).

(i) A peptide represented by the following formula (I):

   NH₂-(Linker)-(X¹₁₋₃)-C-(X²)-(X³)-(X⁴)-(X⁵)-G-(X⁶)-L-(X⁷)-W-C-(X⁸_{1- 3})-(Linker) ··· (I)

   [in formula (I), each (Linker) is independently the same or different linker, or is absent, and the linker is RRRGS (SEQ ID NO: 136), EEGGS (SEQ ID NO: 137), (GSGGS (SEQ ID NO: 138))₁₋₃ or (PEG)₁₋₁₀ (preferably (PEG)₁₋₈ or (PEG)₂₋₁₀, more preferably (PEG)₄),
   X¹ in the number of 1 to 3, X², X³, X⁴, X⁵, X⁶, X⁷, and X⁸ in the number of 1 to 3 are each independently the same or different amino acid residues,
   each of X¹, X², X³ and each X⁸ are each independently the same or different any amino acid residues other than C,
   X⁴ is H, R, S, or D,
   X⁵ is one amino acid residue selected from K, C, D, E, R, V, F, L, 2-aminosuberic acid, Dpr, Orn, AcOrn, AcDab, Dab, Nle, Nva, Tle, Ala(t-Bu), and Cha,
   X⁶ is E, N, R, or D, and
   X⁷ is I or V.

Dab, Tie and Cha mean 2,4-diaminobutanoic acid, tert-leucine and β-cyclohexyl-L-alanine, respectively. Ac and t-Bu mean having an acetyl group and a tert-butyl group, respectively.]

In the formula (I), for the purpose of binding, an amino group may be bound to the end (-COOH) of the C-terminal amino acid of the formula (I) to form a (-C(=O)NH₂) group. In the formula (I), the amino group at the N-terminus may be acetylated. In this case, a Lys residue may be introduced into an appropriate position near the N-terminus in the Linker.

Preferably, the peptides represented by the aforementioned formula (I) include the following peptides:
[1] X¹₁₋₃ is an amino acid sequence shown by (S, G, F, or absent)-(D, G, A, S, P, Hcy, or absent)-(S, D, T, N, E, or R).
[2] X¹₁₋₃ is D, GPD, R, GPR, SPD, GDD, GPS, SDD, RGN, G-Hcy-D, RGP, or GPD.
[3] X¹₁₋₃ is D or GPD.
[4] X² is A, S, or T.
[5] X² is A or T.
[6] X² is A.
[7] X³ is Y or W.
[8] X³ is Y.
[9] X⁴ is H.
[10] X⁵ is one amino acid residue selected from A, R, K, C, D, E, L, 2-aminosuberic acid, Dpr, R, F, 2-aminosuberic acid, Dpr, AcOrn, AcDab, Dab, Nle, Nva, Ala(t-Bu), and Cha.
[11] X⁵ is K, R, AcOrn.
[12] X⁵ is one amino acid residue selected from V, Dab, F, R, L, Nva, Nle, Ala(t-Bu), and Cha.
[13] X⁵ is one amino acid residue selected from F, R, L, Nva, Nle, Ala(t-Bu), and Cha.
[14] X⁵ is one amino acid residue selected from L, Ala(t-Bu), and Cha.
[15] X⁶ is E or N.
[16] X⁶ is E.
[17] X⁷ is V.
[18] X⁸₁₋₃ is (S, T, or D)-(H, G, Y, T, N, D, F, Hey, or absent)-(Y, F, H, M, or absent).
[19] X⁸₁₋₃ is T, TFH, S, SFH, THH, TFY, TYH, or T-Hcy-H.
[20] X⁸₁₋₃ is T or TFH.

The peptide represented by the aforementioned formula (I) may be any one or a combination of two or more of the above conditions, and may be, for example, a peptide satisfying the conditions described below: [8] and [9]; [8] and [17]; [9] and [17]; [8], [9] and [17]; or a combination of these and any one of [10] to [14].

More specifically, the following peptides can be mentioned (X⁵ is the same as above; it may have an NH₂-(Linker)- group at the N-terminus; it may have an -NH₂ group or -(Linker)-NH₂ group at the C-terminus):
1) DCAYHX⁵GELVWCT (SEQ ID NO: 1)
2) GPDCAYHX⁵GELVWCTFH (SEQ ID NO: 2)
3) RCAYHX⁵GELVWCS (SEQ ID NO: 3)
4) GPRCAYHX⁵GELVWCSFH (SEQ ID NO: 4)
5) SPDCAYHX⁵GELVWCTFH (SEQ ID NO: 5)
6) GDDCAYHX⁵GELVWCTFH (SEQ ID NO: 6)
7) GPSCAYHX⁵GELVWCTFH (SEQ ID NO: 7)
8) GPDCAYHX⁵GELVWCSFH (SEQ ID NO: 8)
9) GPDCAYHX⁵GELVWCTHH (SEQ ID NO: 9)
10) GPDCAYHX⁵GELVWCTFY (SEQ ID NO: 10)
11) SPDCAYHX⁵GELVWCTFY (SEQ ID NO: 11)
12) SDDCAYHX⁵GELVWCTFY (SEQ ID NO: 12)
13) RGNCAYHX⁵GQLVWCTYH (SEQ ID NO: 13)
14) G-Hcy-DCAYHX⁵GELVWCT-Hcy-H (SEQ ID NO: 14)
15) RRGPDCAYHX⁵GELVWCTFH (SEQ ID NO: 15)
16) DCTYHX⁵GNLVWCT (SEQ ID NO: 16)
17) DCAYHX⁵GNLVWCT (SEQ ID NO: 17)
18) DCTYHX⁵GELVWCT (SEQ ID NO: 18)
19) DCAWHX⁵GELVWCT (SEQ ID NO: 19)
20) DCTYTX⁵GNLVWCT (SEQ ID NO: 20)
21) DCAYTX⁵GNLVWCT (SEQ ID NO: 21)
22) DCSYTX⁵GNLVWCT (SEQ ID NO: 22)
23) DCTWTX⁵GNLVWCT (SEQ ID NO: 23)
24) DCTYHX⁵GNLVWCT (SEQ ID NO: 24)
25) DCTYRX⁵GNLVWCT (SEQ ID NO: 25)
26) DCTYSX⁵GNLVWCT (SEQ ID NO: 26)
27) DCTYTX⁵GNLVWCT (SEQ ID NO: 27)
28) DCTYTX⁵GELVWCT (SEQ ID NO: 28)
29) DCTYTX⁵GRLVWCT (SEQ ID NO: 29)
30) DCTYTX⁵GDLVWCT (SEQ ID NO: 30)
31) DCTYTX⁵GNLIWCT (SEQ ID NO: 31)
32) DCAYHRGELVWCT (SEQ ID NO: 32)
33) GPDCAYHRGELVWCTFH (SEQ ID NO: 33)
34) RCAYHRGELVWCS (SEQ ID NO: 34)
35) GPRCAYHRGELVWCSFH (SEQ ID NO: 35)
36) SPDCAYHRGELVWCTFH (SEQ ID NO: 36)
37) GDDCAYHRGELVWCTFH (SEQ ID NO: 37)
38) GPSCAYHRGELVWCTFH (SEQ ID NO: 38)
39) GPDCAYHRGELVWCSFH (SEQ ID NO: 39)
40) GPDCAYHRGELVWCTHH (SEQ ID NO: 40)
41) GPDCAYHRGELVWCTFY (SEQ ID NO: 41)
42) SPDCAYHRGELVWCTFY (SEQ ID NO: 42)
43) SDDCAYHRGELVWCTFY (SEQ ID NO: 43)
44) DCTYHRGNLVWCT (SEQ ID NO: 44)
45) DCAYHRGNLVWCT (SEQ ID NO: 45)
46) DCTYHRGELVWCT (SEQ ID NO: 46)
47) DCAWHRGELVWCT (SEQ ID NO: 47)
48) DCTYTNGNLVWCT (SEQ ID NO: 48)
49) DCAYTNGNLVWCT (SEQ ID NO: 49)
50) DCSYTNGNLVWCT (SEQ ID NO: 50)
51) DCTWTNGNLVWCT (SEQ ID NO: 51)
52) DCTYHNGNLVWCT (SEQ ID NO: 52)
53) DCTYRNGNLVWCT (SEQ ID NO: 53)
54) DCTYSNGNLVWCT (SEQ ID NO: 54)
55) DCTYTRGNLVWCT (SEQ ID NO: 55)
56) DCTYTNGELVWCT (SEQ ID NO: 56)
57) DCTYTNGRLVWCT (SEQ ID NO: 57)
58) DCTYTNGDLVWCT (SEQ ID NO: 58)
59) DCTYTNGNLIWCT (SEQ ID NO: 59)

Examples of a peptide bound to a linker include peptides having the following structures.
60) GSGGS-GPDCAYHRGELVWCTFH-NH₂ (SEQ ID NO: 60) (PEG)₄-GPDCAYHRGELVWCTFH-NH₂((PEG)₄-SEQ ID NO: 33-NH₂)
61) GSGGS-DCAYHRGELVWCT-NH₂ (SEQ ID NO: 61) (PEG)₄-DCAYHRGELVWCT-NH₂((PEG)₄-SEQ ID NO: 32-NH₂)

Furthermore, in this embodiment, the "peptide" and the "peptide capable of specifically binding to the Fc region of IgG" include peptides to which a functional group Z is bound. For example, the aforementioned peptide represented by the formula (I) may have a functional group Z bound to the end of (Linker). Examples of such peptides include the following peptides.
62) Acetyl-K(Z)-RRRGS-GPDCAYHKGELVWCTFH-NH₂ (SEQ ID NO: 62)
63) Acetyl-K(Z)-EEGGS-GPDCAYHKGELVWCTFH-NH₂ (SEQ ID NO: 63)
64) Acetyl-K(Z)-(PEG)₄-GPDCAYHKGELVWCTFHNH₂ (Acetyl-K(Z)-(PEG)₄-SEQ ID NO: 64-NH₂)

Alternatively, the following peptides may have a maleimide group, DBCO group, tetrazine group, or TCO group bound to the N-terminus and/or an NH₂ group bound to the C-terminus.
RRRGS-GPDCAYHKGELVWCTFH (SEQ ID NO: 65)
EEGGS-GPDCAYHKGELVWCTFH (SEQ ID NO: 66)
(PEG)₄-GPDCAYHKGELVWCTFH((PEG)₄-SEQ ID NO: 64)

Furthermore, the following peptides can be recited as peptides having a functional group Z bound to the end of (Linker).
67) Acetyl-K(Z)RRRGS-DCAYHKGELVWCT-NH₂ (SEQ ID NO: 67)
68) Acetyl-K(Z)EEGGS-DCAYHKGELVWCT-NH₂ (SEQ ID NO: 68)
69) Acetyl-K(Z)-(PEG)₄-DCAYHKGELVWCT-NH₂ (Acetyl-K(Z)-(PEG)₄-SEQ ID NO: 69-NH₂)

Alternatively, the peptides may be the following peptides having a maleimide group, DBCO group, tetrazine group, or TCO group bound to the N-terminus and/or an NH₂ group bound to the C-terminus.
RRRGS-DCAYHKGELVWCT (SEQ ID NO: 70)
EEGGS-DCAYHKGELVWCT (SEQ ID NO: 71)
(PEG)₄-DCAYHKGELVWCT ((PEG)₄-SEQ ID NO: 69)

(ii) A peptide represented by the following formula (II), or a peptide containing an amino acid sequence in which one or several amino acids are added, deleted, and/or substituted at positions other than X⁹ to X¹⁴ in the amino acid sequence of (II) :

X⁹₁₋₂NMQX¹⁰QX¹⁴RFYEALHDPNLNEEQRNAX¹¹IX¹²SIRDDX¹³-(Linker2)-CONH₂ ··· (II)

[in the formula (II), (Linker2) is (GSGGS)₁₋₃, (SGSGS)₁₋₃, or (PEG)₂₋₁₀-Lys (preferably, (PEG)₄-Lys), SGSGSK, SRRCR, SRRK(Z)R, SRRCRRCRRC, SRRK(Z)RRK(Z)RRK(Z), or (PEG)₁₋₈-Lys (preferably, (PEG)₄-Lys), or absent,
X⁹₁₋₂ is selected from the group consisting of GF, AF, VF, LF, IF, MF, PF, FF, WF, KF, Orn-F, CF, DF, EF, betaalanine-F, 2-aminosuberic acid-F, Dpr-F, NH₂-(PEG)ₙ-CO (wherein n=1-50)-F, F, K, Orn, C, D, E, 2-aminosuberic acid, Dpr, and Acetyl-K,
X¹⁰ is C or Q,
X¹¹ and X¹² are each independently selected from the group consisting of R, H, D, E, S, T, N, Q, Y, and C,
X¹³ is C or P, or absent, and
X¹⁴ is R, C, K, or K(Z)].

The end (-NH₂) of the N-terminal amino acid of the formula (II) may be acetylated to form a (CH₃-C(=O)-NH-) group. In addition, the Cys residue (C) contained in the linker may be bonded to another functional molecule via a maleimide group, where necessary.

Preferably, the following peptides are recited as peptides having the amino acid sequence represented by the aforementioned formula (II).
[21] X⁹ is selected from the group consisting of GF, AF, βAlaF, NH2-(PEG)n-CO (n=2-10)-F, F, K, Orn, C, Dpr, and Acetyl-K.
[22] X⁹ is selected from the group consisting of GF, F, K, and Acetyl-K.
[23] X¹⁰ is Q.
[24] X¹¹ and X¹² are each independently selected from the group consisting of R, H, and E.
[25] X¹¹ is R.
[26] X¹² is R or K(Z) (preferably, Z is azide) .

Specific examples of peptides having the amino acid sequence represented by formula (II) include the following peptides (however, the lysine residues contained therein may be bound to a functional group as necessary):
72) FNMQCQRRFYEALHDPNLNEEQRNARIRSIRDDC (SEQ ID NO: 72)
73) GFNMQCQRRFYEALHDPNLNEEQRNARIRSIRDDC (SEQ ID NO: 73)
74) KNMQCQRRFYEALHDPNLNEEQRNARIRSIRDDC (SEQ ID NO: 74)
75) GFNMQCQKRFYEALHDPNLNEEQRNARIRSIRDDC (SEQ ID NO: 75)
76) KNMQCQKRFYEALHDPNLNEEQRNARIRSIRDDC (SEQ ID NO: 76)
77) FNMQQQRRFYEALHDPNLNEEQRNARIRSIRDD (SEQ ID NO: 77)
78) GKNMQCQRRFYEALHDPNLNEEQRNARIRSIRDDC (SEQ ID NO: 78)
79) Acetyl-FNMQQQRRFYEALHDPNLNEEQRNARIRSIRDDP-SGSGSK-NH₂ (SEQ ID NO: 79)
80) Acetyl-FNMQCQRRFYEALHDPNLNEEQRNARIRSIRDDC-SGSGSK-NH₂ (SEQ ID NO: 80)
81) Acetyl-FNMQQQRRFYEALHDPNLNEEQRNARIRSIRDDP-(PEG)₄-Lys-NH₂ (SEQ ID NO: 81)
72-2) Acetyl-FNMQCQRRFYEALHDPNLNEEQRNARIRSIRDDC-(PEG)₄-Lys-NH₂ (Acetyl-SEQ ID NO: 72-(PEG)₄-Lys-NH₂)
82) FNMQQQCRFYEALHDPNLNEEQRNARIRSIRDD-NH₂ (SEQ ID NO: 82)
83) FNMQQQRRFYEALHDPNLNEEQRNARIRSIRDDC-NH₂ (SEQ ID NO: 83)
84) FNMQQQRRFYEALHDPNLNEEQRNARIRSIRDDP-SRRK(Z)R-NH₂ (SEQ ID NO: 84)
85) FNMQQQRRFYEALHDPNLNEEQRNARIRSIRDDP-SRRCR-NH₂ (SEQ ID NO: 85)
86) FNMQQQCRFYEALHDPNLNEEQRNARICSIRDDP-SRRCRRCRRC-NH₂ (SEQ ID NO: 86),
87) FNMQQQK(Z)RFYEALHDPNLNEEQRNARIK(Z)SIRDDP-SRRK(Z)RRK(Z)RRK(Z)-NH₂ (SEQ ID NO: 87)
88) Acetyl-KNMQQQCRFYEALHDPNLNEEQRNARIRSIRDD-NH₂ (SEQ ID NO: 88)
89) Acetyl-KNMQQQRRFYEALHDPNLNEEQRNARIRSIRDDC-NH₂ (SEQ ID NO: 89)
90) Acetyl-KNMQQQRRFYEALHDPNLNEEQRNARIRSIRDDP-SRRK(Z)R-NH₂ (SEQ ID NO: 90)
91) Acetyl-KNMQQQRRFYEALHDPNLNEEQRNARIRSIRDDP-SRRCR-NH₂ (SEQ ID NO: 91)
92) Acetyl-KNMQQQCRFYEALHDPNLNEEQRNARICSIRDDP-SRRCRRCRRC-NH₂ (SEQ ID NO: 92)
93) Acetyl-KNMQQQK(Z)RFYEALHDPNLNEEQRNARIK(Z)SIRDDP-SRRK(Z)RRK(Z)RRK(Z)-NH₂ (SEQ ID NO: 93)
94) GFNMQQQCRFYEALHDPNLNEEQRNARIRSIRDD-NH₂ (SEQ ID NO: 94)
95) GFNMQQQRRFYEALHDPNLNEEQRNARIRSIRDDC-NH₂ (SEQ ID NO: 95)
96) GFNMQQQRRFYEALHDPNLNEEQRNARIRSIRDDP-SRRK(Z)R-NH₂ (SEQ ID NO: 96)
97) GFNMQQQRRFYEALHDPNLNEEQRNARIRSIRDDP-SRRCR-NH₂ (SEQ ID NO: 97)
98) GFNMQQQCRFYEALHDPNLNEEQRNARICSIRDDP-SRRCRRCRRC-NH₂ (SEQ ID NO: 98)
99) GFNMQQQK(Z)RFYEALHDPNLNEEQRNARIK(Z)SIRDDP-SRRK(Z)RRK(Z)RRK(Z)-NH₂ (SEQ ID NO: 99)
100) FNMQCQZRFYEALHDPNLNEEQRNARIRSIRDDC-NH₂ (SEQ ID NO: 100)
101) Acetyl-KNMQCQZRFYEALHDPNLNEEQRNARIRSIRDDC-NH₂ (SEQ ID NO: 101)
102) GFNMQCQK(Z)RFYEALHDPNLNEEQRNARIRSIRDDC-SRRK(Z)R-NH₂ (SEQ ID NO: 102)
103) FNMQCQK(Z)RFYEALHDPNLNEEQRNARIRSIRDDC-NH₂ (SEQ ID NO: 103)
104) Acetyl-KNMQCQK(Z)RFYEALHDPNLNEEQRNARIRSIRDDC-SRRK(Z)R-NH₂ (SEQ ID NO: 104)
105) GFNMQCQK(Z)RFYEALHDPNLNEEQRNARIRSIRDDC-SRRK(Z)RRK(Z)RRK(Z)-NH₂ (SEQ ID NO: 105)
106) Acetyl-KNMQCQK(Z)RFYEALHDPNLNEEQRNARIRSIRDDC-SRRK(Z)RRK(Z)RRK(Z)-NH₂ (SEQ ID NO: 106)
107) GFNMQCQK(Z)RFYEALHDPNLNEEQRNARIRSIRDDC-SRRK(Z)RRK(Z)RRK(Z)-NH₂ (SEQ ID NO: 107)

(iii) the following peptides:
108) CAWHLGELVWC (SEQ ID NO: 108)
109) DCAWHLGELVWCT (SEQ ID NO: 109)
110) DCAWHLGELVFCT (SEQ ID NO: 110)
111) DCAWHLGELVX¹⁵CT (SEQ ID NO: 111) X¹⁵=1-naphtoyl, 2-naphtoyl, benzyl, or benzothiophene
112) CDCAWHLGELVWCTC (SEQ ID NO: 112)
113) CAYHLGELVWC (SEQ ID NO: 113)
114) DCAYHLGELVWCTF(2-Pya) (SEQ ID NO: 114)
115) Acetyl-(Lys[Azide])RRRGSGPDCAYHKGELVWCTFH-CONH₂) (SEQ ID NO: 115)

The aforementioned peptide may have a reactive functional group (preferably an azide group) bound to the N-terminus or C-terminus (preferably the N-terminus) via a linker as necessary. For example, the peptide may have a reactive functional group (e.g., azide group) at the end of the peptide to which 1 to 3 (preferably 2) glutamic acids are further bound to the N-terminus and/or C-terminus of the peptide. A peptide having an azide group can be linked to another functional molecule having dibenzylcyclooctyne (DBCO), alkyne, or TCO by click reaction. The peptide can also be bound to another functional molecule by other methods known to those skilled in the art, such as the reaction of a maleimide group with a sulfhydryl group.

The protein or peptide may be intramolecularly crosslinked. The protein or peptide may have, for example, one or more (for example, 1-10, 2-8, 4-6, 1, 2, 3, 4, 5, or 6) intramolecular or intermolecular crosslinks. The crosslinks may be thiol bonds, or may be structures in which SH groups are bonded together via the following structure. 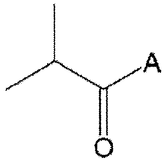 [in the formula, A is a hydrogen atom, a C1-6 alkyl group optionally substituted with a phenyl group or a halogen atom, a phenyl group, or a C1-6 alkyl group.]

For example, the protein may be a protein of 500 Da or more, 1000 Da or more, 2000 Da or more, and/or 500,000 Da or less, 200,000 Da or less, 100,000 Da or less. For example, such proteins may be enzymes, glycoproteins (such as erythropoietin), cytokines, toxins, adjuvants, structural proteins, antibodies, Fc fusion proteins, antibody fragments (such as F(ab')₂, Fab' , Fab, Fab₃, single chain Fv (scFv), (tandem) bispecific single chain Fv (sc(Fv)₂), single chain triple body, nanobody, divalent VHH, pentavalent VHH, minibody, (two chain) diabody, tandem diabody, bispecific tribody, bispecific bibody, dual affinity retargeting molecule (DART), triabody (or tribody), tetrabody (or [sc(Fv)₂]₂), or (scFv-SA)₄) disulfide linked Fv, compact IgG, heavy chain antibody, or polymers thereof). Also, for example, the peptide may be a peptide of 5 to 5000 amino acids, 5 to 1000 amino acids, 5 to 500 amino acids, 5 to 100 amino acids, 5 to 50 amino acids, or 10 to 40 amino acids. Also, the peptide may be either cyclic or linear. Such peptides include vaccines, micro antibodies, and antibacterial peptides.

In this embodiment, X^{m} (m is an integer) represents an amino acid. "X^{m}ₙ" represents that n amino acids X^{m} are bonded, and "X^{m}" with no n represents that one amino acid X^{m} is present. Here, when n is 2 or more, the multiple X^{m} may each independently be the same or different amino acids. When n is "p-q", it represents that p to q amino acids X^{m} are present. Furthermore, in the amino acid sequence described in this embodiment, A is an alanine residue, R is an arginine residue, N is an asparagine residue, D is an aspartic acid residue, C is a cysteine residue, Q is a glutamine residue, E is a glutamic acid residue, G is a glycine residue, H is a histidine residue, I is an isoleucine residue, L is a leucine residue, K is a lysine residue, M is a methionine residue, F is a phenylalanine residue, P is a proline residue, S is a serine residue, T is a threonine residue, W is a tryptophan residue, Y is a tyrosine residue, and V is a valine residue. In addition, Hcy is homocysteine, Dpr is diaminopropionic acid, Orn is an ornithine residue, βAla is a β-alanine residue, Dab is a 2,4-diaminobutyric acid residue, Nle is a norleucine residue, Nva is a norvaline residue, Tle is a tert-leucine residue, Ala(t-Bu) is a tert-butylalanine residue, and Cha is a cyclohexylalanine residue. In addition, the amino group in the residue having an amino group in the side chain (lysine residue, ornithine residue, 2,4-diaminobutyric acid residue) may be acetylated as necessary. In the present embodiment, the acetylated form of natural and artificial amino acids may be described with the prefix Ac in the amino acid symbols described above. It is understood that the acetylated form may be included even when Ac is not indicated, unless such interpretation is not particularly inconsistent. In this embodiment, K(Z) means a functional group-bound lysine residue, and preferably K(Z) is K(Azide). K(Azide) shows an azide-bound lysine residue.

The amino group in the protein or peptide that binds to the crosslinking reagent may be an amino group possessed by a lysine residue, cysteine residue, aspartic acid residue, glutamic acid residue, 2-aminosuberic acid, diaminopropionic acid, or arginine residue present in the aforementioned protein or peptide, an amino group of an amino acid at position 1, or an amino group introduced into the carbonyl group terminal, and is preferably an amino group possessed by a lysine residue, cysteine residue, aspartic acid residue, glutamic acid residue, 2-aminosuberic acid, diaminopropionic acid, or arginine residue, or an amino group of an amino acid at position 1.

The crosslinking reagents include disuccinimidyl glutarate (DSG), disuccinimidyl suberate (DSS), dimethyl adipimidate dihydrochloride (DMA), dimethyl pimelimidate dihydrochloride (DMP), dimethyl suberimidate dihydrochloride (DMS), dimethyl 3,3'-dithiobispropionimidate dihydrochloride (DTBP), and dithiobis(succinimidyl propionate) (DSP).

Throughout this embodiment, a "reactive functional group" or a group represented by "Z" refers to a group that can react with and bond to a peptide, protein, nucleic acid, or small molecule drug under relatively mild conditions. The reactive functional groups include maleimide, thiol or protected thiol, alcohol, acrylate, acrylamide, amine or protected amine, carboxylic acid or protected carboxylic acid, azide, alkyne including cycloalkyne, 1,3-dienes including cyclopentadiene and furan, alpha-halocarbonyl, N-hydroxysuccinimidyl, N-hydroxysulfosuccinimidyl, nitrophenyl ester, carbonate, dibenzocyclooctyne (DBCO), tetrazine, methyltetrazine (MTZ), transcyclooctene (TCO), azide, carboxy, tosyl, amino, epoxy, acyl, isothiocyanate, isocyanate, acyl azide, NHS ester, acid chloride, aldehyde, glyoxal, epoxide, oxirane, carbonate, arylating agent, imidoester, carbodiimide, acid anhydride, haloacetyl, alkyl halide, maleimide, aziridine, acryloyl derivative, arylating agent, diazoalkane, diazoacetyl compound, carbonyl, ketone, carbodiimide, epoxide, oxirane, carbonyldiimidazole, N,N'-disuccinimidyl carbonate, N-hydroxysuccinimidyl chloroformate, alkyl halide, isocyanate, hydrazine, Schiff base, reductive amination product, Mannich condensation product, diazonium derivative, Mannich condensation product, iodination product, aryl azide, halogenated aryl azide, benzophenone, diazo compound, and diazirine derivative.

The method according to the present embodiment may include purifying by size exclusion chromatography after binding an amino group in a protein or peptide to a crosslinking agent. As the carrier for size exclusion chromatography, resins that can be used with organic solvents, such as Sephadex, Sepharose, Superdex, Superose, Sephacryl, Biogel, Cellulofine, Toyopearl, and Sephadex LH-20, and that can be used for open column, low pressure, and high pressure chromatography, can be used. The pore size of the carrier can be preferably 30 to 2000 Å, more preferably 100 to 300 Å. The particle size of the carrier may be preferably 5 to 500 µm, more preferably 20 to 120 µm. The column size is preferably a column with a gel volume of 10 to 1000 mL or more for an open column, and a column with a gel volume of 3 to 100 mL or more for a packed column.

Elution conditions for size exclusion chromatography are preferably 4°C to room temperature, and an eluent may be an aqueous solution of a volatile strong acid such as 0.01-1% TFA or HCl, or a volatile weak acid such as 1-30% formic acid or acetic acid. It is also possible to use a mixture of 1-100% volatile and hydrating organic solvent (CH₃CN, MetOH, EtOH, propanol, acetone, etc.)/water, containing a volatile strong acid such as 0.01-1% TFA or HCl.

The second embodiment of the present invention encompasses the following technical ideas.
[1] A method for producing an antibody-labeling reagent, comprising reacting a protein or peptide with the aforementioned crosslinking reagent in the presence of a base having a stronger degree of basicity than pyridine to bond an amino group in the protein or peptide with the crosslinking reagent.
[2] A method for producing an antibody-labeling reagent of [1], wherein the aforementioned base has a pKa greater than 5.2.
[3] The method for producing an antibody-labeling reagent of [2], wherein the aforementioned base has a pKa of 14 or less.
[4] The method for producing an antibody-labeling reagent of [1], wherein the aforementioned base is triethylamine, N,N-diisopropylethylamine, diethylamine, or monomethylamine.
[5] The method for producing an antibody-labeling reagent of [1], wherein the aforementioned base is triethylamine.
[6] The method for producing an antibody-labeling reagent of any of [1] to [5], wherein the concentration of the aforementioned base is 0.01 to 5%.
[7] The method for producing an antibody-labeling reagent of any of [1] to [6], wherein the aforementioned protein or peptide is a peptide capable of specifically binding to the Fc region of IgG.
[8] The method for producing an antibody-labeling reagent of [7], wherein the amino acid sequence of the aforementioned peptide capable of specifically binding to an Fc region of IgG is an amino acid sequence shown in any one of SEQ ID NOs: 1 to 115.
[9] The method for producing an antibody-labeling reagent of any of [1] to [8], wherein the amino group is an amino group possessed by a lysine residue, a cysteine residue, an aspartic acid residue, a glutamic acid residue, a 2-aminosuberic acid, a diaminopropionic acid, or an arginine residue present in the aforementioned protein or peptide, an amino group of an amino acid at position 1, or an amino group introduced at the carbonyl group terminal.
[10] The method for producing an antibody-labeling reagent of any of [1] to [9], wherein the aforementioned crosslinking reagent is selected from the group consisting of disuccinimidyl glutarate (DSG), disuccinimidyl suberate (DSS), dimethyl adipimidate dihydrochloride (DMA), dimethyl pimelimidate dihydrochloride (DMP), dimethyl suberimidate dihydrochloride (DMS), dimethyl 3,3'-dithiobispropionimidate dihydrochloride (DTBP), and dithiobis(succinimidyl propionic acid) (DSP).
[11] A method for producing an antibody-labeling reagent of any of [1] to [10], further comprising purifying by size exclusion chromatography.

The present invention is described in more detail in the following by using examples, but the scope of the present invention is not limited thereby.

### [Examples]

### (Example 1)

The terms, abbreviations, and definitions used in this example are shown in Table 1.

**[Table 1]**

| term and abbreviation | definition |
|---|---|
| IgGBP | IgG Binding Peptide: IgG-binding peptide |
| MPA002 | azide-conjugated IgGBP |
| MPA003 | succinimidyl glutarate-modified MPA002 |
| 3arm DOTA peptide | 3arm DOTA-conjugated IgGBP |
| DSG | disuccinimidyl glutarate |
| NMM | N-methylmorpholine |
| TEA | triethylamine |

The IgGBP used was GPDCAYHKGELVWCTFH (SEQ ID NO: 134, two Cys in the molecule form SS bonds, and the C-terminus is amidated).

MPA002 is the following structure produced according to the method described in WO2017/217347, and is an IgGBP to which ethyl azide is bound via a linker structure having diglycolic acid and eight ethylene glycol groups at the N-terminus.

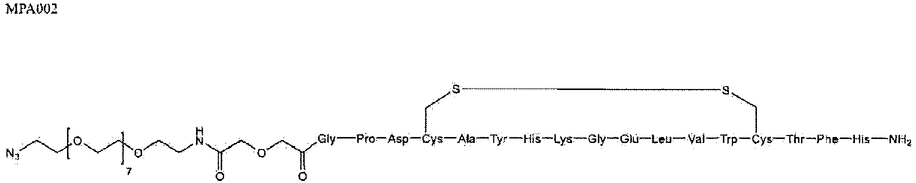

The 3armDOTA peptide is the following structure and was produced according to the method described in WO2017/217347.

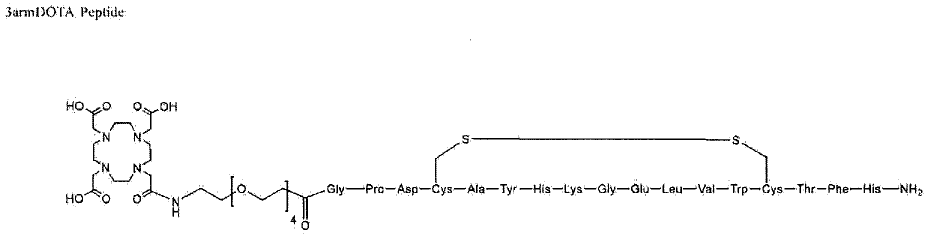

### Experimental Example 1-1: Production and evaluation of antibody-modification linker using IgG-binding peptide with N-terminus modified with chelating agent

### (Comparative Example 1-1)

The 3armDOTA peptide (about 1 mg) was dissolved in DMSO (13 µL) to give an about 30 mmol/L peptide solution. To this peptide solution were added 13 µL (10 equivalents relative to 3armDOTA peptide) of about 300 mmol/L DSG dissolved in acetonitrile and 0.2 µL (5 equivalents relative to 3armDOTA peptide) of pyridine to a final concentration of about 0.5%, and the reaction was performed at 50°C for 6 hr.

After the reaction, 1 µL of the solution was diluted with 0.1% formic acid/DMSO solution (99 µL) and analyzed under the test conditions shown in Table 2.

**[Table 2]**

| test conditions | | |
|---|---|---|
| detector: ultraviolet absorption spectrophotometer (measurement wavelength: 280 nm) | | |
| column: ACQUITY UPLC CSH C18 1.7 µm 2.1×100 mm | | |
| column temperature: 60°C | | |
| mobile phase A: water/trifluoroacetic acid mixed solution (1000:1) | | |
| mobile phase B: acetonitrile/trifluoroacetic acid mixed solution (1000:1) | | |
| mobile phase delivery: | | |
| time (min) after injecting | mobile phase A (vol%) | mobile phase B (vol%) |
| 0 - 10.0 | 80→60 | 20→40 |
| 10.0 - 10.5 | 60→10 | 40→90 |
| 10.5 - 14.5 | 10 | 90 |
| 14.5 - 15.0 | 10→80 | 90→20 |
| 15.0 - 20.0 | 80 | 20 |

| | | |
|---|---|---|
| flow: 0.3 mL/min area measurement range: 20 min injection volume: 5 µL | | |

The amount of unreacted 3armDOTA peptide (IgG binding peptide), hydrolyzate, and succinimidyl glutarate-modified 3armDOTA peptide (antibody-modification linker) was calculated as area percentage (unit: %). The results are shown in Table 3.

**[Table 3]**

| type of base | Pyridine |
|---|---|
| pKa of base | 5.25 |
| number of base equivalents | 5 |
| reaction solvent | DMSO/ACN |
| reaction time (min) | 360 |
| reaction temperature (°C) | 50 |
| IgG binding peptide | 1.3 |
| hydrolyzate | 18.0 |
| antibody-modification linker | 52.1 |

It was found that a large amount of hydrolyzate was produced that was difficult to remove by purification.

### (Example 1-1) Production of antibody-modification linker by using 3armDOTA peptide

The 3armDOTA peptide (about 1 mg) was dissolved in DMSO (13 µL) to give an about 30 mmol/L peptide solution. To this peptide solution were added 13.3 µL (10 equivalents relative to 3armDOTA peptide) of 300 mmol/L DSG dissolved in acetonitrile and 0.21 µL (5 equivalents relative to 3armDOTA peptide) of NMM, and the reaction was performed at -20°C for 1 hr. Similarly, samples were prepared by reacting at 20°C for 1 hr, 20°C for 1.3 hr, 5°C for 3 hr, or -10°C for 3 hr.

After the reaction, 1 µL of each solution was diluted with DMSO (49 µL) and analyzed under the test conditions shown in Table 2.

The amount of unreacted 3armDOTA peptide (IgG binding peptide), hydrolyzate, and succinimidyl glutarate-modified 3armDOTA peptide (antibody-modification linker) was calculated as area percentage (unit: %). The results are shown in Table 4.

**[Table 4]**

| | | | | | |
|---|---|---|---|---|---|
| type of base | NMM | ← | ← | ← | ← |
| pKa of base | 7.38 | ← | ← | ← | ← |
| number of base equivalents | 5 | ← | ← | ← | ← |
| reaction solvent | DMSO/ACN | ← | ← | ← | ← |
| reaction time (min) | 60 | 80 | 180 | 180 | 60 |
| reaction temperature (°C) | 20 | 20 | 5 | -10 | -20 |
| IgG binding peptide | 0.1 | 0.5 | 0.7 | 4.13 | 8.15 |
| hydrolyzate | 14.7 | 14.8 | 13.8 | 6.98 | 4.85 |
| antibody-modification linker | 58.5 | 60.7 | 63.3 | 75.40 | 70.21 |

In all cases, the amount of hydrolyzate was reduced without reducing the amount of the antibody-modification linker produced. Pyridine, used as a base in Comparative Example 1-1, is classified as a weak base (pKa: 5.25). On the other hand, NMM, used as a base in Example 1-1, has a pKa of 7.38 and has higher basicity than pyridine. From the above results, it was found that a base with stronger basicity than pyridine may be used to reduce the amount of hydrolyzate while maintaining the yield at the same level as before, and that the reaction proceeds sufficiently at reaction temperatures below room temperature.

### Experimental Example 1-2: Production and evaluation of antibody-modification linker using IgG-binding peptide with N-terminus modified with azide

### (Comparative Example 1-2)

MPA002 (about 1 mg) was dissolved in DMSO (20 µL) to give an about 19 mmol/L peptide solution. To this peptide solution was added 20 µL of a solution of DSG in acetonitrile (500 mmol/L), then 0.27 µL of pyridine (6.5 equivalents relative to MPA002) was added to a final concentration of 0.5%, and the reaction was performed at 50°C for 3 hr.

After the reaction, 1 µL of the solution was diluted with DMSO (49 µL) and analyzed under the following test conditions.

**[Table 5]**

| test conditions | | |
|---|---|---|
| detector: ultraviolet absorption spectrophotometer (measurement wavelength: 280 nm) | | |
| column: ACQUITY UPLC CSH C18 1.7 um 2.1×100 mm | | |
| column temperature: 60°C | | |
| mobile phase A: water/trifluoroacetic acid mixed solution (1000:1) | | |
| mobile phase B: acetonitrile/trifluoroacetic acid mixed solution (1000:1) | | |
| mobile phase delivery: | | |
| time (min) after injecting | mobile phase A (vol%) | mobile phase B (vol%) |
| 0 - 10.0 | 72→67 | 28→33 |
| 10.0 - 10.5 | 67→10 | 33→90 |
| 10.5 - 14.5 | 10 | 90 |
| 14.5 - 15.0 | 10→72 | 90→28 |
| 15.0 - 20.0 | 72 | 28 |

| | | |
|---|---|---|
| flow: 0.3 mL/min area measurement range: 14.5 min injection volume: 5 µL | | |

The amount of unreacted MPA002 (IgG binding peptide), hydrolyzate, and succinimidyl glutarate-modified MPA002 (MPA003,antibody-modification linker) was calculated as area percentage (unit: %). The results are shown in Table 6.

**[Table 6]**

| type of base | Pyridine |
|---|---|
| pKa of base | 5.25 |
| number of base equivalents | 6.5 |
| reaction solvent | DMSO/ACN |
| reaction time (min) | 180 |
| reaction temperature (°C) | 50 |
| IgG binding peptide | 3.19 |
| hydrolyzate | 5.87 |
| antibody-modification linker | 72.49 |

### (Examples 1-2 to 1-3) Study of reaction temperature and/or base equivalent using MPA002

MPA002 (about 1 mg) was dissolved in DMSO (20 µL) to give an about 19 mmol/L peptide solution. To this peptide solution were added 20 µL of a solution of DSG acetonitrile solution (500 mmol/L), and then 0.13 µL (3 equivalents relative to MPA002), 0.21 µL (5 equivalents relative to MPA002), or 0.42 µL (10 equivalents relative to MPA002) of NMM, and the reaction was performed at 10°C, 0°C, -10°C, -20°C, or -30°C for 1, 2, or 3 hr.

After the reaction, 1 µL of the solution was diluted with DMSO (49 µL) and analyzed under the test conditions shown in Table 5.

The amount of unreacted MPA002 (IgG binding peptide), hydrolyzate, and succinimidyl glutarated MPA002 (MPA003, antibody-modification linker) was calculated as area percentage (unit: %). The study results of the reaction temperature are shown in Table 7, and the study results of the base equivalent are shown in Table 8.

In both cases, the amount of hydrolyzate produced was smaller than that in Comparative Example 1-2 shown in Table 6.

**[Table 7]**

| | | | | | | | | |
|---|---|---|---|---|---|---|---|---|
| type of base | NMM | ← | ← | ← | ← | ← | ← | ← |
| reaction temperature (°C) | -30 | ← | -20 | ← | ← | -10 | ← | 0 |
| number of base equivalents | 5 | ← | ← | ← | ← | ← | ← | ← |
| reaction time (min) | 120 | 180 | 60 | 120 | 180 | 60 | 120 | 60 |
| IgG binding peptide | 14.72 | 8.50 | 10.45 | 2.00 | 0.41 | 5.55 | 0.54 | 0.92 |
| hydrolyzate | 2.71 | 2.96 | 2.95 | 3.89 | 4.71 | 3.42 | 4.50 | 4.45 |
| antibody-modification linker | 73.12 | 78.52 | 76.57 | 83.34 | 83.46 | 80.92 | 83.94 | 83.77 |

**[Table 8]**

| | | | | | | | | | | | |
|---|---|---|---|---|---|---|---|---|---|---|---|
| type of base | NMM | ← | ← | ← | ← | ← | ← | ← | ← | ← | ← |
| reaction temperature (°C) | -30 | ← | ← | -20 | ← | -10 | ← | ← | 0 | ← | 10 |
| number of base equivalents | 10 | ← | ← | ← | ← | ← | 3 | ← | ← | ← | ← |
| reaction time (min) | 120 | ← | 180 | 60 | 120 | 60 | 120 | 180 | 60 | 120 | ← |
| IgG binding peptide | 7.52 | 2.49 | 0.99 | 0.83 | 0.00 | 0.34 | 11.11 | 5.21 | 11.52 | 2.65 | 3.45 |
| hydrolyzate | 3.01 | 3.34 | 3.65 | 3.58 | 4.56 | 3.87 | 3.29 | 4.16 | 3.08 | 4.68 | 4.80 |
| antibody-modification linker | 79.09 | 82.36 | 84.79 | 84.45 | 83.81 | 83.23 | 75.26 | 80.57 | 74.55 | 82.09 | 81.40 |

Furthermore, when 0.27 µL of NMM (6.5 equivalents relative to MPA002) was added and the reaction was performed at 30°C for 1, 2, or 3 hr, the area percentage (%) of each component was higher for the hydrolyzate than the results in Tables 7 and 8, and lower for the antibody-modification linker than the results in Tables 7 and 8.

### (Example 1-4) Examination of scale-up

Whether the method according to the first embodiment of the present invention can be applied even when the reaction scale is increased was examined.

MPA002 (about 20 mg) was dissolved in DMSO (400 µL) to give an about 19 mmol/L peptide solution. To this peptide solution were added 400 µL (26 equivalents relative to MPA002) of a solution of DSG in acetonitrile (500 mmol/L), and then 2.51 µL of NMM (3 equivalents relative to MPA002), and the reaction was performed at 0°C for 1 or 2 hr.

Meanwhile, MPA002 (about 1 mg) was dissolved in DMSO (20 µL) to give an about 19 mmol/L peptide solution. To this peptide solution were added 20 µL of a solution of DSG in acetonitrile (500 mmol/L), and then 0.13 µL of NMM (3 equivalents relative to MPA002), and the reaction was performed at 0°C for 1 or 2 hr.

After the reaction, 1 µL of each solution was diluted with DMSO (49 µL) and analyzed under the conditions shown in Table 5.

The amount of unreacted MPA002 (IgG binding peptide), hydrolyzate, and succinimidyl glutarated MPA002 (MPA003, antibody-modification linker) was calculated as area percentage (unit: %). The results are shown in Table 9.

**[Table 9]**

| | | | | |
|---|---|---|---|---|
| reaction temperature (°C) | 0°C | ← | ← | ← |
| IgG-binding peptide amount | 1 mg | ← | 20 mg | ← |
| reaction time (min) | 60 | 120 | 60 | 120 |
| IgG-binding peptide | 11.52 | 2.65 | 15.09 | 4.94 |
| hydrolyzate | 3.08 | 4.68 | 3.64 | 4.86 |
| antibody-modification linker | 74.55 | 82.09 | 71.24 | 79.13 |

All showed good results, and it was shown that the method according to the first embodiment of the present invention can be applied even when the reaction scale is increased.

### (Example 1-5) Study of base type

As shown in the above example, the present inventors found that the excellent conditions were to use NMM, which is a stronger base than pyridine, and to lower the reaction temperature. Therefore, the reaction was performed using TEA (pKa: 10.75), which is a stronger base than NMM, and by further lowering the reaction temperature.

MPA002 (about 1 mg) was dissolved in DMSO (20 µE) to give an about 19 mmol/L peptide solution. To this peptide solution were added 20 µL (26 equivalents relative to MPA002) of a solution of DSG in acetonitrile (500 mmol/L), and then 0.16 µL of TEA (3 equivalents relative to MPA002), and the reaction was performed at -20°C for 2 or 3 hr.

After the reaction, 1 µL of each solution was diluted with DMSO (49 µL) and analyzed under the conditions shown in Table 5.

The amount of unreacted MPA002 (IgG binding peptide), hydrolyzate, and succinimidyl glutarated MPA002 (MPA003, antibody-modification linker) was calculated as area percentage (unit: %). The results are shown in Table 10. As a result of using NMM as the base, data for reaction temperatures of -20°C and reaction times of 120 and 180 min (Table 7 in Examples 1-2) are also included.

**[Table 10]**

| | | | | |
|---|---|---|---|---|
| type of base | NMM | ← | TEA | ← |
| pKa of base | 7.38 | ← | 10.72 | ← |
| number of base equivalents | 5 | ← | 3 | ← |
| reaction time (min) | 120 | 180 | 120 | 180 |
| reaction temperature (°C) | -20 | ← | ← | ← |
| IgG binding peptide | 2.00 | 0.41 | 12.37 | 8.50 |
| hydrolyzate | 3.89 | 4.71 | 3.68 | 4.00 |
| antibody-modification linker | 83.34 | 83.46 | 73.44 | 76.86 |

It was confirmed that the amount of hydrolyzate was reduced in both cases, and that the antibody-modification linker, which was the target product, could be synthesized in a sufficient amount.

From the above results, it was found that, when producing an antibody-modification linker, it is preferable to perform binding to a crosslinking agent by using a base having a stronger basicity than pyridine,
(1) at a higher reaction temperature with a smaller base equivalent for a shorter reaction time,
(2) at a lower reaction temperature with a larger base equivalent for a longer reaction time.

### (Example 2)

### (Example 2-1) Preparation of IgG-binding peptide CCAP reagent

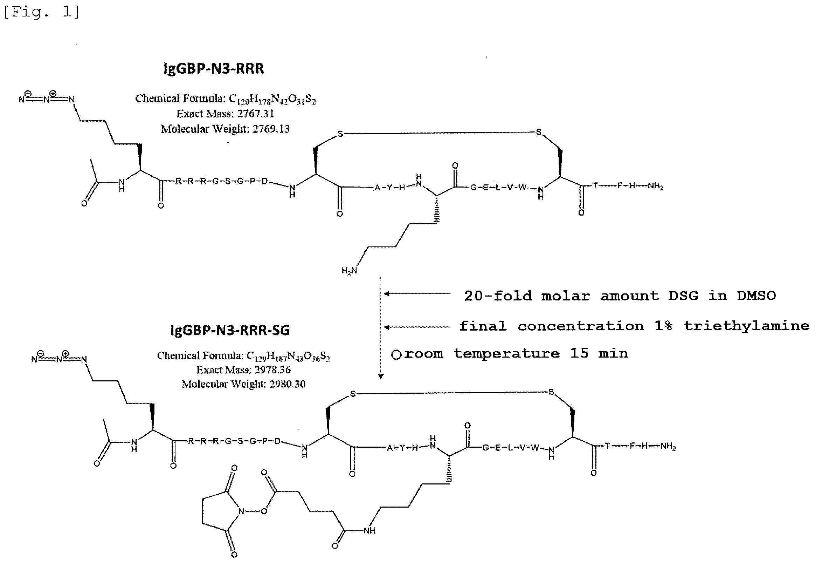

The scheme for the modification of IgG-BP using DSG by an improved method is shown in Fig. 1. IgG-BP (IgGBP-N3-RRR:Acetyl-(Lys[Azide])RRRGSGPDCAYHKGELVWCTFH-CONH₂) (SEQ ID NO: 115) was reacted with 20 times the molar concentration of DSG in DMSO containing 1% triethylamine at room temperature for 15 min. Samples before and 15 min after the addition of DSG were subjected to UPLC analysis under the following conditions.

That is, 1 nmol of sample was injected into an ACQUITY UPLC BEH C18 column (1.7 µm, 2.1 × 100 mm) connected to a UPLC (Waters) coupled with ACQUITY SQD, and eluted with a gradient of 10% to 40% acetonitrile containing 0.1% formic acid at a flow rate of 0.2 ml/min. The column temperature was 35°C. The elution was monitored by measuring the absorbance at 280 nm.

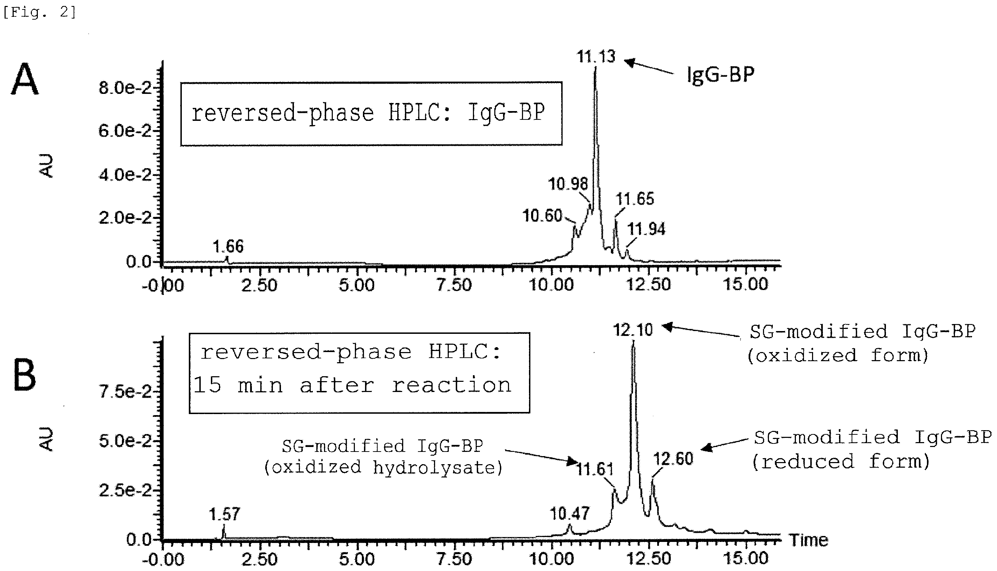

The chromatograms of the UPLC analysis before and 15 min after the start of the reaction are shown in Fig. 2A and Fig. 2B, respectively. In the UPLC pattern after 15 min (Fig. 2B), the peak of IgG-BP (elution time 11.13 min) that existed before the reaction disappeared, and a new peak at elution time 12.10 min was obtained. Mass spectrometry of this peak gave a mass value of 2978.34, which matched the mass of the target CCAP reagent (theoretical MW: 2978.36) in which IgG-BP had been SG-modified. Therefore, it was found that IgG-BP was SG-modified. This extremely rapid SG modification was not observed in the conventional pyridine/DMSO system. Even though a small amount of SG-modified hydrolyzate was observed at 11.61 min and a small amount of SS-reduced product of the SG-modified CCAP reagent at 12.60 min, no other large peaks of side reactions were observed, suggesting that side reactions were avoided by the short reaction time.

### (Example 2-2) Purification of IgG-binding peptide CCAP reagent

To remove excess DSG from the reaction solution, size exclusion chromatography was performed using a Sephadex G-10 column equilibrated with 0.1% TFA. That is, the reaction mixture (about 0.8 mL) diluted 5-fold with 0.1% TFA was injected at a flow rate of 2.0 mL/min into a Sephadex G-10 column (25 × 600 mm) connected to an NGC chromatograph (BioRad), and the eluate was fractionated and collected. The elution was monitored by measuring the absorbance at 220 and 280 nm.

The purity of the peptides collected from the flow-through fraction was checked by reversed-phase UPLC. Analysis was performed using an ACQUITY UPLC BEH C18 column connected to a UPLC (Waters) coupled with an ACQUITY SQD, as described above.

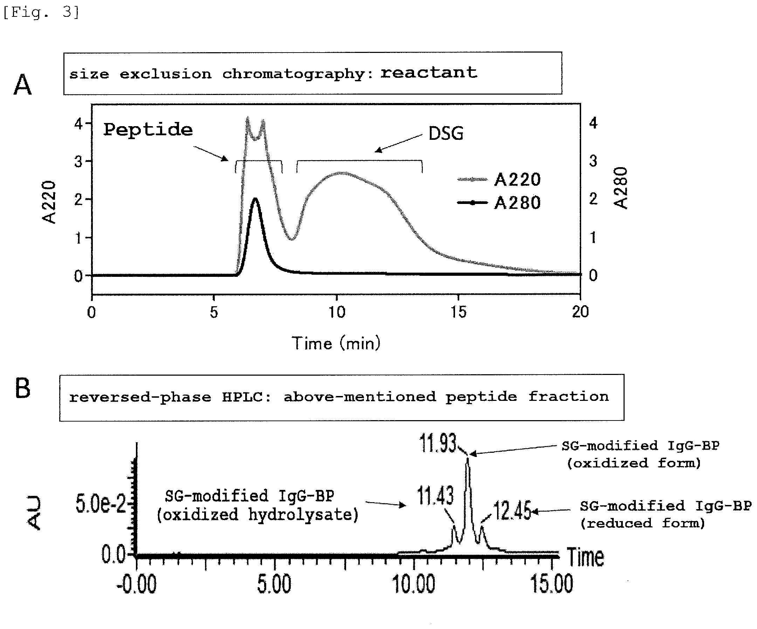

The results of size exclusion chromatography are shown in Fig. 3A. Since the SG-modified IgG-BP was collected in the flow-through fraction, and DSG was eluted later, it was found that the two could be separated clearly. The purity of the peptides collected from the flow-through fraction was checked by reversed-phase UPLC, and the results are shown in Fig. 3B. Although hydrolysates of SG-modified IgG-BP (oxidized form) and SG-modified IgG-BP (reduced form) were observed in addition to the desired SG-modified IgG-BP (oxidized form), a reagent with a purity of about 78% was obtained, and when it was actually reacted with IgG, no problem was found with the reactivity. The SG-modified IgG-BP (reduced form) identified as an impurity was contained from the raw material stage (peak at 11.65 min in Fig. 2A), and it is considered that a CCAP reagent with high purity can be synthesized in the future by using a high-purity raw material (oxidized IgG-BP). In addition, although detailed data is not shown, the yield of the CCAP reagent of IgG-BP by the SG modification according to the conventional method was about 50% calculated from the raw peptide, whereas it increased to about 65% by the improved method.

### (Example 2-3) Preparation of Z33 peptide CCAP reagent

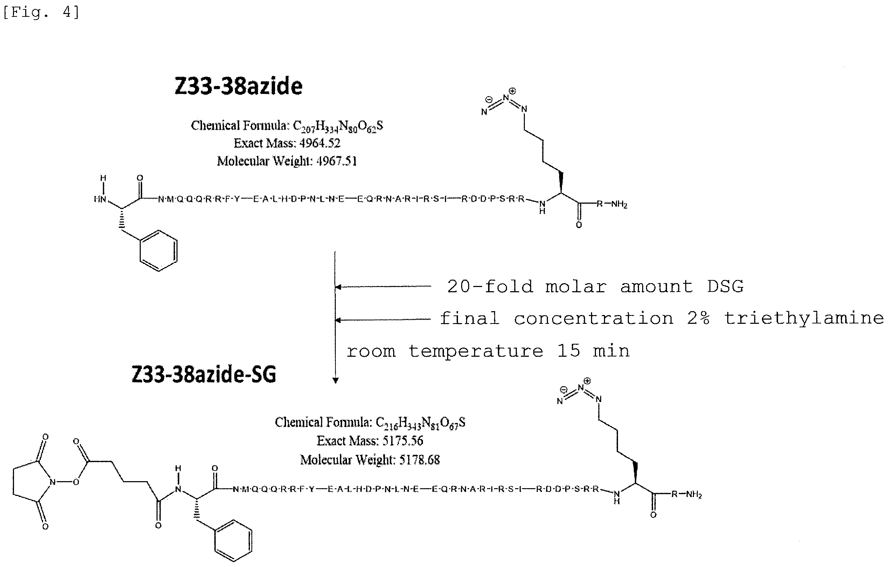

As shown in Fig. 4, a Z33-based CCAP reagent was also prepared by the improved method. The Z33-38azide peptide was subjected to reversed-phase UPLC under the following conditions. That is, the sample was injected into an ACQUITY UPLC BEH C18 column (1.7 um, 2.1 × 100 mm) connected to a UPLC (Waters) coupled with an ACQUITY SQD, and eluted with a gradient of 10% to 40% acetonitrile containing 0.1% formic acid at a flow rate of 0.2 ml/min. The column temperature was 35°C, and the elution was monitored by measuring the absorbance at 280 nm.

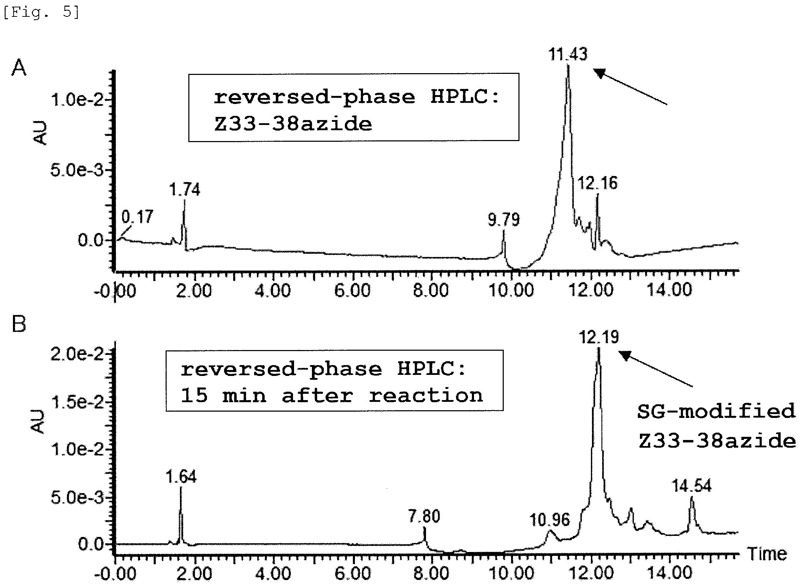

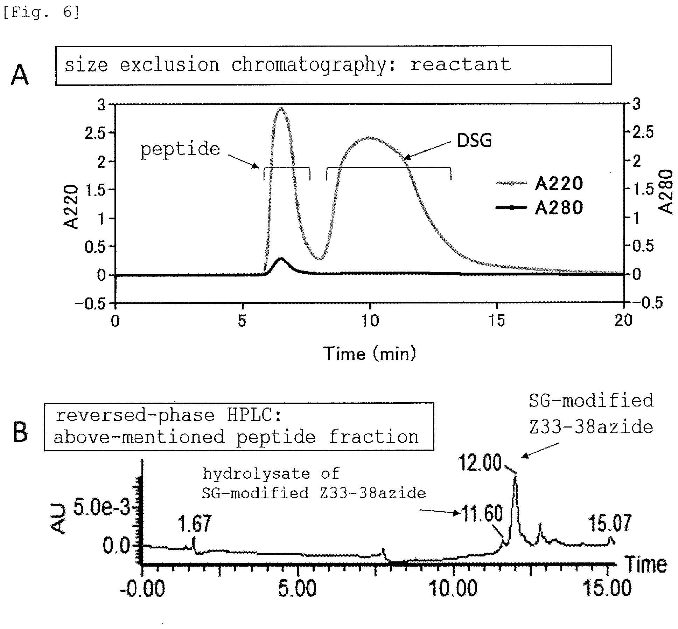

As shown in Fig. 5A, mass spectrometry revealed that Z33-38azide was the peak seen at 11.43 min. This peptide was reacted with DSG under the same conditions as IgG-BP in Fig. 1 (room temperature, 15 min), and then subjected to UPLC. The raw material disappeared, and the production of the desired SG-modified Z33-38azide was confirmed as a new peak at 12.19 min (Fig. 5A). This reaction product was diluted 5-fold with 0.1% TFA (about 0.4 mL), applied to a Sephadex G-10 column (Fig. 6A), and the flow-through fraction was collected as CCAP reagent. The purity of the obtained CCAP reagent was analyzed by reversed-phase UPLC.

The results are shown in Fig. 6B. The chromatogram showed that the purity of the desired SG-modified Z33-38azide was 89%. In addition, the yield of the Z33 CCAP reagent from the conventional SG modification was about 30% calculated from the raw peptide, whereas the improved method increased the yield to about 50%.

### (Example 2-4) Study of reaction time and base equivalent by using IgGBP-N3-RRR

IgGBP-N3-RRR was dissolved in DMSO (25 µl) to give a peptide solution of about 14 mM. To this peptide solution were added 10 µl of 1 M DSG DMSO solution (final concentration about 280 mM), and then 0.21 µl (3 equivalents relative to peptide), 0.35 µl (5 equivalents relative to peptide), 0.46 µl (6.5 equivalents relative to peptide) or 0.61 µl (8.76 equivalents relative to peptide) of TEA, and the reaction was performed at room temperature for 15 min, 1 hr or 3 hr, respectively.

After the reaction, 2 µL of each solution was diluted with 10% formic acid (18 µL) and analyzed in the same manner as in Example 2-1.

When 3, 5, 6.5 or 8.76 equivalents of TEA relative to peptide were reacted with the peptide for 15 min, 1 hr or 3 hr, the desired SG-modified IgG-BP (oxidized form) was obtained in high yields.

Since the amount of hydrolyzate tended to increase with increasing reaction time, the present invention exerts a more superior effect by a reaction in a short time.

In addition, the amount of hydrolyzate tended to increase with increasing number of equivalents of TEA.

The above-mentioned embodiments are intended to describe the present invention and do not limit the scope of the present invention. In other words, the scope of the present invention is indicated by the claims, not by the embodiments. Various modifications made within the scope of the claims and within the scope of the meaning of the invention equivalent thereto are considered to be within the scope of the present invention.

The contents of all publications, including patents and patent application specifications, mentioned herein are incorporated herein by reference to the same extent as if expressly set forth in their entireties.

This application is based on Japanese Patent Application Nos. 2022-037732 and 2022-038459, filed on March 11, 2022, respectively, in Japan, the contents of which are incorporated in full herein.

The present invention is useful for the modification of Fc-containing molecules, the production of modified Fc-containing molecules and Fc-containing molecule modifying reagents, and research reagents.

## Claims

1. A method for producing an Fc-containing molecule modifying reagent, comprising reacting a protein or peptide with a crosslinking agent in the presence of a base having a stronger basicity than pyridine, thereby binding an amino group in the protein or peptide to the crosslinking agent.

2. The method for producing an Fc-containing molecule modifying reagent according to claim 1, wherein the protein or peptide is an IgG-binding peptide, the crosslinking agent has a succinimide group capable of binding to a terminal amino group of a side chain of an amino acid residue constituting the IgG-binding peptide, the base is a tertiary amine, and the reaction is performed at room temperature or a lower temperature.

3. The method for producing an Fc-containing molecule modifying reagent according to claim 2, wherein the tertiary amine is a tertiary amine having a pKa of 6.0 or more and 15.0 or less.

4. The method for producing an Fc-containing molecule modifying reagent according to claim 2, wherein the tertiary amine is a tertiary amine having a pKa of 6.0 or more and 8.0 or less.

5. The method for producing an Fc-containing molecule modifying reagent according to claim 2, wherein the reaction is performed in the presence of a molar amount of the tertiary amine of 2 equivalents to 20 equivalents based on the molar amount of the IgG-binding peptide.

6. The method for producing an Fc-containing molecule modifying reagent according to claim 2, wherein the reaction is performed at 5°C or lower.

7. The method for producing an Fc-containing molecule modifying reagent according to claim 2, wherein the reaction is performed at -5°C or below.

8. The method for producing an Fc-containing molecule modifying reagent according to claim 2, wherein the crosslinking agent is DSG (disuccinimidyl glutarate) or DSS (disuccinimidyl suberate).

9. The method for producing an Fc-containing molecule modifying reagent according to claim 2, wherein the IgG-binding peptide comprises an amino acid sequence consisting of 13 to 17 amino acid residues and represented by the following formula I:
(X)₁₋₃-C-(X)₂-H- (Xaa1) -G- (Xaa2) -L-V-W-C-(X)₁₋₃ (I)
wherein each X is independently any amino acid residue other than cysteine,
C is a cysteine residue,
H is a histidine residue,
Xaa1 is a lysine residue,
G is a glycine residue,
Xaa2 is a glutamic acid residue, glutamine residue or
asparagine residue,
L is a leucine residue,
V is a valine residue, and
W is a tryptophan residue.

10. The method for producing an Fc-containing molecule modifying reagent according to claim 2, wherein the N-terminus of the IgG-binding peptide is modified with a chelating agent capable of chelating a radioactive metal or an atomic group capable of a click reaction.

11. The method for producing an Fc-containing molecule modifying reagent according to claim 1, wherein the base has a pKa greater than 5.2.

12. The method for producing an Fc-containing molecule modifying reagent according to claim 11, wherein the base has a pKa of 14 or less.

13. The method for producing an Fc-containing molecule modifying reagent according to claim 1, wherein the base is triethylamine, N,N-diisopropylethylamine, diethylamine, or monomethylamine.

14. The method for producing an Fc-containing molecule modifying reagent according to claim 1, wherein the base is triethylamine.

15. The method for producing an Fc-containing molecule modifying reagent according to claim 1, wherein the concentration of the base is 0.01 to 5%.

16. The method for producing an Fc-containing molecule modifying reagent according to claim 1, wherein the protein or peptide is a peptide capable of specifically binding to the Fc region of IgG.

17. The method for producing an Fc-containing molecule modifying reagent according to claim 16, wherein the amino acid sequence of the peptide capable of specifically binding to an Fc region of IgG is an amino acid sequence shown in any one of SEQ ID NOs: 1 to 115.

18. The method for producing an Fc-containing molecule modifying reagent according to claim 17, wherein the amino group is an amino group possessed by a lysine residue, a cysteine residue, an aspartic acid residue, a glutamic acid residue, a 2-aminosuberic acid, a diaminopropionic acid, or an arginine residue present in the protein or peptide, an amino group of an amino acid at position 1, or an amino group introduced at the carbonyl group terminal.

19. The method for producing an Fc-containing molecule modifying reagent according to claim 1, wherein the crosslinking agent is selected from the group consisting of disuccinimidyl glutarate (DSG), disuccinimidyl suberate (DSS), dimethyl adipimidate dihydrochloride (DMA), dimethyl pimelimidate dihydrochloride (DMP), dimethyl suberimidate dihydrochloride (DMS), dimethyl 3,3'-dithiobispropionimidate dihydrochloride (DTBP), and dithiobis(succinimidyl propionic acid) (DSP).

20. The method for producing an Fc-containing molecule modifying reagent according to claim 1, further comprising purifying by size exclusion chromatography.

21. A method for producing a conjugate including a linker modification step of performing the method for producing an Fc-containing molecule modifying reagent according to any one of claims 1 to 20 to obtain an Fc-containing molecule modifying reagent, and a conjugation step of mixing the Fc-containing molecule modifying reagent with an Fc-containing molecule, and reacting the Fc-containing molecule modifying reagent with an amino acid at a side chain terminal of an amino acid constituting the Fc-containing molecule to obtain a conjugate of the Fc-containing molecule modifying reagent and the Fc-containing molecule.
